# EUROPEAN PATENT SPECIFICATION

(11) **EP 4 351 687 B1**
(45) Date of publication and mention of the grant of the patent: **08.07.2026**
(21) Application number: 22809952.9
(22) Date of filing: 16.05.2022
(51) Int. Cl.: A61M 16/06, A61M 16/20, A61M 16/08

(54) **PATIENT INTERFACE WITH DETACHABLE, SEALED CHASSIS**
PATIENTENSCHNITTSTELLE MIT ABNEHMBAREM, VERSIEGELTEM CHASSIS
INTERFACE PATIENT AVEC CHÂSSIS AMOVIBLE ET SCELLÉ

(30) Priority: 24.05.2021 US 202163192132 P
(43) Date of publication of application: 17.04.2024
(73) Proprietor: ResMed Pty Ltd, Bella Vista, New South Wales 2153 (AU)
(72) Inventor: EVES, Matthew, Bella Vista, New South Wales 2153 (AU)
(74) Representative: Vossius & Partner Patentanwälte Rechtsanwälte mbB
(86) International application number: PCT/AU2022/050464
(87) International publication number: WO 2022/246498

(56) References cited:
- WO-A1-2005/063327
- WO-A1-2014/015382
- WO-A1-2015/070289
- WO-A1-2020/208523
- WO-A2-2016/054692
- US-B2- 10 744 293

## Description

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

This application claims priority to U.S. Provisional Application No. 63/192,132, filed May 24, 2021.

### 1 BACKGROUND OF THE TECHNOLOGY

### 1.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use.

### 1.2 DESCRIPTION OF THE RELATED ART

### 1.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See "Respiratory Physiology", by John B. West, Lippincott Williams & Wilkins, 9th edition published 2012.

### 1.2.2 Therapies

Various respiratory therapies, such as Continuous Positive Airway Pressure (CPAP) therapy, Non-invasive ventilation (NIV), Invasive ventilation (IV), and High Flow Therapy (HFT) have been used to treat one or more of the above respiratory disorders.

### 1.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

### 1.2.3 Respiratory Therapy Systems

These respiratory therapies may be provided by a respiratory therapy system or device. Such systems and devices may also be used to screen, diagnose, or monitor a condition without treating it.

A respiratory therapy system may comprise a Respiratory Pressure Therapy Device (RPT device), an air circuit, a humidifier, a patient interface, an oxygen source, and data management.

### 1.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 cmH₂O relative to ambient pressure. For other forms of therapy, such as the delivery of oxygen, the patient interface may not include a seal sufficient to facilitate delivery to the airways of a supply of gas at a positive pressure of about 10 cmH₂O. For flow therapies such as nasal HFT, the patient interface is configured to insufflate the nares but specifically to avoid a complete seal. One example of such a patient interface is a nasal cannula.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some masks suffer from being one or more of obtrusive, aesthetically undesirable, costly, poorly fitting, difficult to use, and uncomfortable especially when worn for long periods of time or when a patient is unfamiliar with a system. Wrongly sized masks can give rise to reduced compliance, reduced comfort and poorer patient outcomes. Masks designed solely for aviators, masks designed as part of personal protection equipment (e.g. filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy. This is even more so if the mask is to be worn during sleep.

CPAP therapy is highly effective to treat certain respiratory disorders, provided patients comply with therapy. If a mask is uncomfortable, or difficult to use a patient may not comply with therapy. Since it is often recommended that a patient regularly wash their mask, if a mask is difficult to clean (e.g., difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep form a distinct field.

### 1.2.3.1.1 Seal-forming structure

Patient interfaces may include a seal-forming structure. Since it is in direct contact with the patient's face, the shape and configuration of the seal-forming structure can have a direct impact the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface, a seal-forming structure may comprise a single element that surrounds both nares in use. Such single element may be designed to for example overlay an upper lip region and a nasal bridge region of a face. In one form of patient interface a seal-forming structure may comprise an element that surrounds a mouth region in use, e.g. by forming a seal on a lower lip region of a face. In one form of patient interface, a seal-forming structure may comprise a single element that surrounds both nares and a mouth region in use. These different types of patient interfaces may be known by a variety of names by their manufacturer including nasal masks, full-face masks, nasal pillows, nasal puffs and oro-nasal masks.

A seal-forming structure that may be effective in one region of a patient's face may be inappropriate in another region, e.g. because of the different shape, structure, variability and sensitivity regions of the patient's face. For example, a seal on swimming goggles that overlays a patient's forehead may not be appropriate to use on a patient's nose.

Certain seal-forming structures may be designed for mass manufacture such that one design fit and be comfortable and effective for a wide range of different face shapes and sizes. To the extent to which there is a mismatch between the shape of the patient's face, and the seal-forming structure of the mass-manufactured patient interface, one or both must adapt in order for a seal to form.

One type of seal-forming structure extends around the periphery of the patient interface, and is intended to seal against the patient's face when force is applied to the patient interface with the seal-forming structure in confronting engagement with the patient's face. The seal-forming structure may include an air or fluid filled cushion, or a moulded or formed surface of a resilient seal element made of an elastomer such as a rubber. With this type of seal-forming structure, if the fit is not adequate, there will be gaps between the seal-forming structure and the face, and additional force will be required to force the patient interface against the face in order to achieve a seal.

Another type of seal-forming structure incorporates a flap seal of thin material positioned about the periphery of the mask so as to provide a self-sealing action against the face of the patient when positive pressure is applied within the mask. Like the previous style of seal forming portion, if the match between the face and the mask is not good, additional force may be required to achieve a seal, or the mask may leak. Furthermore, if the shape of the seal-forming structure does not match that of the patient, it may crease or buckle in use, giving rise to leaks.

Another type of seal-forming structure may comprise a friction-fit element, e.g. for insertion into a naris, however some patients find these uncomfortable.

Another form of seal-forming structure may use adhesive to achieve a seal. Some patients may find it inconvenient to constantly apply and remove an adhesive to their face.

A range of patient interface seal-forming structure technologies are disclosed in the following patent applications, assigned to ResMed Limited: WO 1998/004,310; WO 2006/074,513; WO 2010/135,785.

One form of nasal pillow is found in the Adam Circuit manufactured by Puritan Bennett. Another nasal pillow, or nasal puff is the subject of US Patent 4,782,832 (Trimble et al.), assigned to Puritan-Bennett Corporation.

ResMed Limited has manufactured the following products that incorporate nasal pillows: SWIFTTM nasal pillows mask, SWIFTTM II nasal pillows mask, SWIFTTM LT nasal pillows mask, SWIFTTM FX nasal pillows mask and MIRAGE LIBERTYTM full-face mask. The following patent applications, assigned to ResMed Limited, describe examples of nasal pillows masks: International Patent Application WO2004/073778 (describing amongst other things aspects of the ResMed Limited SWIFTTM nasal pillows), US Patent Application 2009/0044808 (describing amongst other things aspects of the ResMed Limited SWIFTTM LT nasal pillows); International Patent Applications WO 2005/063328 and WO 2006/130903 (describing amongst other things aspects of the ResMed Limited MIRAGE LIBERTYTM full-face mask); International Patent Application WO 2009/052560 (describing amongst other things aspects of the ResMed Limited SWIFTTM FX nasal pillows).

WO2005/063327 discloses a mask system which includes frame, cushion, cushion clip, swivel elbow, headgear and/or headgear clips configured for use in a clinical or hospital environment. The frame can be flexible and deform with predetermined and/or repeated application of force.

WO2005/063327 discloses a patient interface to deliver a flow of air at a positive pressure with respect to ambient air pressure to an entrance to the patient's airways. The patient interface includes a frame assembly and a cushion assembly configured to removably and repeatably connect to the frame assembly. The frame assembly and the cushion assembly form at least part of a plenum chamber pressurizable to a therapeutic pressure. The cushion assembly comprises a one-piece construction including a seal-forming structure configured to form a seal with a region of a patient's face surrounding the entrance to the patient's airways and a frame connection structure configured to removably and repeatably connect the cushion assembly to the frame assembly. The seal-forming structure comprises a first elastomeric material and the frame connection structure comprises a second elastomeric material, the first elastomeric material comprising a lower durometer or hardness than the second elastomeric material.

WO2016/054692 discloses a mask apparatus for a respiratory treatment can permit delivery of breathable gas to a user. In one example, the mask may employ a rigid frame and a flexible cushion to form a seal for both mouth and nose.

### 1.2.3.1.2 Positioning and stabilising

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

### 1.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include a CPAP device and a ventilator.

### 1.2.3.3 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

### 1.2.3.4 Humidifier

Delivery of a flow of air without humidification may cause drying of airways. The use of a humidifier with an RPT device and the patient interface produces humidified gas that minimizes drying of the nasal mucosa and increases patient airway comfort. In addition, in cooler climates, warm air applied generally to the face area in and about the patient interface is more comfortable than cold air.

### 1.2.3.5 Data Management

There may be clinical reasons to obtain data to determine whether the patient prescribed with respiratory therapy has been "compliant", e.g. that the patient has used their RPT device according to one or more "compliance rules". One example of a compliance rule for CPAP therapy is that a patient, in order to be deemed compliant, is required to use the RPT device for at least four hours a night for at least 21 of 30 consecutive days. In order to determine a patient's compliance, a provider of the RPT device, such as a health care provider, may manually obtain data describing the patient's therapy using the RPT device, calculate the usage over a predetermined time period, and compare with the compliance rule. Once the health care provider has determined that the patient has used their RPT device according to the compliance rule, the health care provider may notify a third party that the patient is compliant.

There may be other aspects of a patient's therapy that would benefit from communication of therapy data to a third party or external system.

Existing processes to communicate and manage such data can be one or more of costly, time-consuming, and error-prone.

### 1.2.3.6 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

The vent may comprise an orifice and gas may flow through the orifice in use of the mask. Many such vents are noisy. Others may become blocked in use and thus provide insufficient washout. Some vents may be disruptive of the sleep of a bed partner 1100 of the patient 1000, e.g. through noise or focussed airflow.

ResMed Limited has developed a number of improved mask vent technologies. See International Patent Application Publication No. WO 1998/034,665; International Patent Application Publication No. WO 2000/078,381; US Patent No. 6,581,594; US Patent Application Publication No. US 2009/0050156; US Patent Application Publication No. 2009/0044808.

**Table of noise of prior masks (ISO 17510-2:2007, 10 cmH2O pressure at 1m)**

| Mask name | Mask type | A-weighted sound power level dB(A) (uncertainty) | A-weighted sound pressure dB(A) (uncertainty) | Year (approx.) |
|---|---|---|---|---|
| Glue-on (*) | nasal | 50.9 | 42.9 | 1981 |
| ResCare standard (*) | nasal | 31.5 | 23.5 | 1993 |
| ResMed MirageTM (*) | nasal | 29.5 | 21.5 | 1998 |
| ResMed UltraMirageT M | nasal | 36 (3) | 28 (3) | 2000 |
| ResMed Mirage ActivaTM | nasal | 32 (3) | 24 (3) | 2002 |
| ResMed Mirage MicroTM | nasal | 30 (3) | 22 (3) | 2008 |
| ResMed MirageTM SoftGel | nasal | 29 (3) | 22 (3) | 2008 |
| ResMed MirageTM FX | nasal | 26 (3) | 18 (3) | 2010 |
| ResMed Mirage SwiftTM (*) | nasal pillows | 37 | 29 | 2004 |
| ResMed Mirage SwiftTM II | nasal pillows | 28 (3) | 20 (3) | 2005 |
| ResMed Mirage SwiftTM LT | nasal pillows | 25 (3) | 17 (3) | 2008 |
| ResMed AirFit P10 | nasal pillows | 21 (3) | 13 (3) | 2014 |

| | | | | |
|---|---|---|---|---|
| (* one specimen only, measured using test method specified in ISO 3744 in CPAP mode at 10 cmH2O) | | | | |

Sound pressure values of a variety of objects are listed below

| Object | A-weighted sound pressure dB(A) | Notes |
|---|---|---|
| Vacuum cleaner: Nilfisk | 68 | ISO 3744 at 1m distance |
| Walter Broadly Litter Hog: B+ Grade | | |
| Conversational speech | 60 | 1m distance |
| Average home | 50 | |
| Quiet library | 40 | |
| Quiet bedroom at night | 30 | |
| Background in TV studio | 20 | |

### 2 BRIEF SUMMARY OF THE TECHNOLOGY

The invention is defined in the independent claim 1. Preferred embodiments are matter of the dependent claims.

The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

An aspect of the present technology is directed to a patient interface that comprises: a plenum chamber having a first connection portion; a seal-forming structure constructed and arranged to seal with a region of the patient's face; a chassis constructed from a flexible material, a second connection portion positioned on the chassis and configured to releasably connect to the first connection portion, and the first connection portion and the second connection portion, when connected, being more rigid than the flexible material; a sealing lip to seal between the plenum chamber and the chassis; a positioning and stabilising structure; and a vent structure.

An aspect of the present technology is directed to a patient interface that comprises: a plenum chamber pressurisable to a therapeutic pressure, the plenum chamber having a plenum chamber opening, and the plenum chamber having a first connection portion positioned proximal to the plenum chamber opening; a seal-forming structure constructed and arranged to seal with a region of the patient's face; a chassis constructed from a flexible material, a second connection portion positioned on the chassis and configured to releasably connect to the first connection portion, and the first connection portion and the second connection portion, when connected, being more rigid than the flexible material; a sealing lip positioned radially around the plenum chamber opening to seal between the plenum chamber and the chassis; a positioning and stabilising structure to provide a force to hold the seal-forming structure in a therapeutically effective position on the patient's head; and a vent structure having a plurality of holes to allow a continuous flow of gases exhaled by the patient from an interior of the plenum chamber to ambient throughout the patient's respiratory cycle.

An aspect of the present technology is directed to a patient interface that comprises: a plenum chamber pressurisable to a therapeutic pressure of at least 6 cmH₂O above ambient air pressure, the plenum chamber having a plenum chamber opening, and the plenum chamber having a first connection portion positioned proximal to the plenum chamber opening; a seal-forming structure constructed and arranged to seal with a region of the patient's face surrounding an entrance to the patient's airways, said seal-forming structure having a hole therein such that the flow of air at said therapeutic pressure is delivered to at least an entrance to the patient's nares, the seal-forming structure being joined to the plenum chamber to maintain said therapeutic pressure in the plenum chamber throughout the patient's respiratory cycle in use; a chassis constructed from a flexible material and including an inlet port sized and structured to receive a flow of air at the therapeutic pressure and direct the flow of air into the plenum chamber through the plenum chamber opening for breathing by a patient, a second connection portion positioned on the chassis and configured to releasably connect to the first connection portion, and the first connection portion and the second connection portion, when connected, being more rigid than the flexible material; a sealing lip positioned radially around the plenum chamber opening to seal between the plenum chamber and the chassis; a positioning and stabilising structure to provide a force to hold the seal-forming structure in a therapeutically effective position on the patient's head, the positioning and stabilising structure comprising a tie use; and a vent structure having a plurality of holes to allow a continuous flow of gases exhaled by the patient from an interior of the plenum chamber to ambient throughout the patient's respiratory cycle, said vent structure being sized and shaped to maintain the therapeutic pressure in the plenum chamber in use; wherein the patient interface is configured to leave the patient's mouth uncovered, or if the seal-forming structure is configured to seal around the patient's nose and mouth, the patient interface is configured to allow the patient to breath from ambient in the absence of a flow of pressurised air through the plenum chamber inlet port.

In examples of the aspects in the three preceding paragraphs, (a) the flexible material may be a textile, (b) the first connection portion may be one of a hook material and a loop material and the second connection portion may be the other of the hook material and the loop material, (c) the first connection portion may be positioned radially outward of the plenum chamber opening, (d) the second connection portion may be positioned radially inward of a peripheral edge of the chassis, (e) the sealing lip may be fixed to the chassis, (f) the sealing lip may be fixed to the plenum chamber, (g) the sealing lip may be positioned radially outward of the first connection portion and the second connection portion, (h) the sealing lip may be positioned radially inward of the first connection portion and the second connection portion, (i) the first connection portion and the second connection portion may be continuous around the plenum chamber and the chassis, respectively, (j) one of the first connection portion and the second connection portion may comprise a plurality of discontinuous portions positioned around the plenum chamber and the chassis, respectively, and the other of the first connection portion and the second connection portion may be continuous around the plenum chamber and the chassis, respectively, (k) each of the first connection portion and the second connection portion may comprise a plurality of discontinuous portions positioned around the plenum chamber and the chassis, respectively, and the plurality of discontinuous portions of each of the first connection portion and the second connection portion may be positioned at corresponding positions around the plenum chamber and the chassis, respectively, to connect to one another, (1) the plurality of discontinuous portions of each of the first connection portion and the second connection portion may be positioned asymmetrically around the plenum chamber and the chassis, respectively, (m) the plurality of discontinuous portions of each of the first connection portion and the second connection portion may be positioned symmetrically around the plenum chamber and the chassis, respectively, (n) the sealing lip, in an undeformed state, may have a dimension extending from the plenum chamber or the chassis in a direction that is orthogonal to a surface to which the sealing lip is attached, and the dimension may be greater than a combined thickness of the first connection portion and the second connection portion, (o) the chassis may include the vent structure, (p) an elbow may be configured to rotatably connect to the chassis at the inlet port at a first end and to an air circuit at a second end to direct the flow of air into the plenum chamber, (q) the elbow may include the vent structure, (r) the sealing lip may be constructed from an elastomeric material, (s) the sealing lip may be constructed from silicone, (t) the plenum chamber and the seal-forming structure may be formed in one piece from a homogeneous material, (t) the plenum chamber and the seal-forming structure may be formed in one piece from a textile, (u) the plenum chamber and the seal-forming structure may be formed in one piece from silicone, (v) the sealing lip may be integrally molded to the plenum chamber, (w) the sealing lip may be integrally molded to the chassis, (x) the sealing lip may be radially continuous, (y) two inlet ports may be positioned on the chassis, the positioning and stabilising structure may comprise a pair of conduits, each configured to connect to a corresponding one of the inlet ports to direct the flow of air into the plenum chamber, (z) the chassis may comprise a pair of tie attachment structures and the positioning and stabilising structure may comprise a pair of ties, each of the ties being configured to attach to a corresponding one of the tie attachment structures to connect the positioning and stabilising structure to the chassis, (aa) the first connection portion may be joined to the plenum chamber with adhesive, (bb) the plenum chamber may be overmolded to the first connection portion, (cc) the first connection portion may be molded into the plenum chamber, (dd) the sealing lip may be positioned such that an increase in pressure within the plenum chamber increases a sealing force of the sealing lip between the plenum chamber and the chassis, (ee) the second connection portion may be positioned on the chassis with a gap between a peripheral edge of the chassis and an outer peripheral edge of the second connection portion, (ff) the seal-forming structure may be configured so as not to enter the patient's mouth in use, (gg) the seal-forming structure may be configured so that the seal-forming structure does not extend internally of the patient's airways in use, (hh) the seal-forming structure may be configured so as not extend to below a mental protuberance region of the patient in use, (ii) the seal-forming structure and the plenum chamber may be configured so as not to cover the patient's eyes in use, (jj) the seal-forming structure may comprise a nasal hole configured to direct the flow of air into the patient's nares during use, (kk) the seal-forming structure may comprise an oral hole configured to direct the flow of air into the patient's mouth during use, (11) the sealing lip may be cantilevered from the chassis or the plenum chamber, and/or (mm) the sealing lip may be fixed to the plenum chamber or the chassis at a first end and may be free at a second end and the sealing lip may be curved between the first end and the second end.

An aspect of the present technology is directed to a patient interface that comprises: a plenum chamber having a first connection portion; a seal-forming structure constructed and arranged to seal with a region of the patient's face; a chassis having a second connection portion configured to releasably attach to the first connection portion, and the chassis having a tie attachment structure; a sealing lip to seal between the plenum chamber and the chassis; a positioning and stabilising structure; and a vent structure.

An aspect of the present technology is directed to a patient interface that comprises: a plenum chamber pressurisable to a therapeutic pressure, the plenum chamber having a plenum chamber opening, and the plenum chamber having a first connection portion positioned proximal to the plenum chamber opening; a seal-forming structure constructed and arranged to seal with a region of the patient's face; a chassis having a second connection portion configured to releasably attach to the first connection portion, and the chassis having a tie attachment structure; a sealing lip positioned radially around the plenum chamber opening to seal between the plenum chamber and the chassis; a positioning and stabilising structure comprising a tie to attach to the tie attachment structure and provide a force to hold the seal-forming structure in a therapeutically effective position on the patient's head; and a vent structure having a plurality of holes to allow a continuous flow of gases exhaled by the patient from an interior of the plenum chamber to ambient throughout the patient's respiratory cycle.

An aspect of the present technology is directed to a patient interface that comprises: a plenum chamber pressurisable to a therapeutic pressure of at least 6 cmH₂O above ambient air pressure, the plenum chamber having a plenum chamber opening, and the plenum chamber having a first connection portion positioned proximal to the plenum chamber opening; a seal-forming structure constructed and arranged to seal with a region of the patient's face surrounding an entrance to the patient's airways, said seal-forming structure having a hole therein such that the flow of air at said therapeutic pressure is delivered to at least an entrance to the patient's nares, the seal-forming structure being joined to the plenum chamber to maintain said therapeutic pressure in the plenum chamber throughout the patient's respiratory cycle in use; a chassis including an inlet port sized and structured to receive a flow of air at the therapeutic pressure and direct the flow of air into the plenum chamber through the plenum chamber opening for breathing by a patient, the chassis having a second connection portion configured to releasably attach to the first connection portion, and the chassis having a tie attachment structure; a sealing lip positioned radially around the plenum chamber opening to seal between the plenum chamber and the chassis; a positioning and stabilising structure comprising a tie to attach to the tie attachment structure and provide a force to hold the seal-forming structure in a therapeutically effective position on the patient's head; and a vent structure having a plurality of holes to allow a continuous flow of gases exhaled by the patient from an interior of the plenum chamber to ambient throughout the patient's respiratory cycle, said vent structure being sized and shaped to maintain the therapeutic pressure in the plenum chamber in use; wherein the patient interface is configured to leave the patient's mouth uncovered, or if the seal-forming structure is configured to seal around the patient's nose and mouth, the patient interface is configured to allow the patient to breath from ambient in the absence of a flow of pressurised air through the plenum chamber inlet port.

In examples of the aspects in the three preceding paragraphs, (a) the flexible material may be a textile, (b) the first connection portion may be one of a hook material and a loop material and the second connection portion may be the other of the hook material and the loop material, (c) the first connection portion may be positioned radially outward of the plenum chamber opening, (d) the second connection portion may be positioned radially inward of a peripheral edge of the chassis, (e) the sealing lip may be fixed to the chassis, (f) the sealing lip may be fixed to the plenum chamber, (g) the sealing lip may be positioned radially outward of the first connection portion and the second connection portion, (h) the sealing lip may be positioned radially inward of the first connection portion and the second connection portion, (i) the first connection portion and the second connection portion may be continuous around the plenum chamber and the chassis, respectively, (j) one of the first connection portion and the second connection portion may comprise a plurality of discontinuous portions positioned around the plenum chamber and the chassis, respectively, and the other of the first connection portion and the second connection portion may be continuous around the plenum chamber and the chassis, respectively, (k) each of the first connection portion and the second connection portion may comprise a plurality of discontinuous portions positioned around the plenum chamber and the chassis, respectively, and the plurality of discontinuous portions of each of the first connection portion and the second connection portion may be positioned at corresponding positions around the plenum chamber and the chassis, respectively, to connect to one another, (1) the plurality of discontinuous portions of each of the first connection portion and the second connection portion may be positioned asymmetrically around the plenum chamber and the chassis, respectively, (m) the plurality of discontinuous portions of each of the first connection portion and the second connection portion may be positioned symmetrically around the plenum chamber and the chassis, respectively, (n) the sealing lip, in an undeformed state, may have a dimension extending from the plenum chamber or the chassis in a direction that is orthogonal to a surface to which the sealing lip is attached, and the dimension may be greater than a combined thickness of the first connection portion and the second connection portion, (o) the chassis may include the vent structure, (p) an elbow may be configured to rotatably connect to the chassis at the inlet port at a first end and to an air circuit at a second end to direct the flow of air into the plenum chamber, (q) the elbow may include the vent structure, (r) the sealing lip may be constructed from an elastomeric material, (s) the sealing lip may be constructed from silicone, (t) the plenum chamber and the seal-forming structure may be formed in one piece from a homogeneous material, (t) the plenum chamber and the seal-forming structure may be formed in one piece from a textile, (u) the plenum chamber and the seal-forming structure may be formed in one piece from silicone, (v) the sealing lip may be integrally molded to the plenum chamber, (w) the sealing lip may be integrally molded to the chassis, (x) the sealing lip may be radially continuous, (y) two inlet ports may be positioned on the chassis, the positioning and stabilising structure may comprise a pair of conduits, each configured to connect to a corresponding one of the inlet ports to direct the flow of air into the plenum chamber, (z) the first connection portion may be joined to the plenum chamber with adhesive, (aa) the plenum chamber may be overmolded to the first connection portion, (bb) the first connection portion may be molded into the plenum chamber, (cc) the sealing lip may be positioned such that an increase in pressure within the plenum chamber increases a sealing force of the sealing lip between the plenum chamber and the chassis, (dd) the second connection portion may be positioned on the chassis with a gap between a peripheral edge of the chassis and an outer peripheral edge of the second connection portion, (ee) the seal-forming structure may be configured so as not to enter the patient's mouth in use, (ff) the seal-forming structure may be configured so that the seal-forming structure does not extend internally of the patient's airways in use, (gg) the seal-forming structure may be configured so as not extend to below a mental protuberance region of the patient in use, (hh) the seal-forming structure and the plenum chamber may be configured so as not to cover the patient's eyes in use, (ii) the seal-forming structure may comprise a nasal hole configured to direct the flow of air into the patient's nares during use, (jj) the seal-forming structure may comprise an oral hole configured to direct the flow of air into the patient's mouth during use, (kk) the sealing lip may be cantilevered from the chassis or the plenum chamber, and/or (11) the sealing lip may be fixed to the plenum chamber or the chassis at a first end and may be free at a second end and the sealing lip may be curved between the first end and the second end.

An aspect of the present technology is directed to a patient interface that comprises: a cushion assembly configured to contact and seal against a patient's face and including a first connection portion; a chassis including a second connection portion configured to releasably connect to the first connection portion to join the chassis and cushion assembly; a sealing lip fixed to and extending from one of the cushion assembly or the chassis and configured to be deformed by contact with the other of the cushion assembly or the chassis before the first connection portion and the second connection portion are connected to seal between cushion assembly and the chassis; a positioning and stabilising structure to provide a force to hold the seal-forming structure in a therapeutically effective position on the patient's head, the positioning and stabilising structure comprising a tie; and a vent structure having a plurality of holes to allow a continuous flow of gases exhaled by the patient from an interior of the cushion assembly to ambient throughout the patient's respiratory cycle.

In examples of the aspect of the preceding paragraph: (a) the cushion assembly may comprise: a plenum chamber pressurisable to a therapeutic pressure of at least 6 cmH₂O above ambient air pressure; and a seal-forming structure constructed and arranged to seal with a region of the patient's face surrounding an entrance to the patient's airways, said seal-forming structure having a hole therein such that the flow of air at said therapeutic pressure is delivered to at least an entrance to the patient's nares, the seal-forming structure being joined to the plenum chamber to maintain said therapeutic pressure in the plenum chamber throughout the patient's respiratory cycle in use, (b) the chassis may include an inlet port sized and structured to receive a flow of air at the therapeutic pressure and direct the flow of air into the plenum chamber through the plenum chamber opening for breathing by the patient, (c) the vent structure may be sized and shaped to maintain the therapeutic pressure in the plenum chamber in use, (d) the plenum chamber may have a plenum chamber opening, (e) the patient interface may be configured to leave the patient's mouth uncovered, or if the seal-forming structure is configured to seal around the patient's nose and mouth, the patient interface may be configured to allow the patient to breath from ambient in the absence of a flow of pressurised air through the plenum chamber inlet port, (f) the chassis may be constructed from a flexible material, (g) the flexible material may be a textile, (h) the first connection portion may be one of a hook material and a loop material and the second connection portion may be the other of the hook material and the loop material, (i) the first connection portion may be positioned radially outward of the plenum chamber opening, (j) the second connection portion may be positioned radially inward of a peripheral edge of the chassis, (k) the sealing lip may be fixed to the chassis, (1) the sealing lip may be fixed to the plenum chamber, (m) the sealing lip may be positioned radially outward of the first connection portion and the second connection portion, (n) the sealing lip may be positioned radially inward of the first connection portion and the second connection portion, (o) the first connection portion and the second connection portion may be continuous around the plenum chamber and the chassis, respectively, (p) one of the first connection portion and the second connection portion may comprise a plurality of discontinuous portions positioned around the plenum chamber and the chassis, respectively, and the other of the first connection portion and the second connection portion may be continuous around the plenum chamber and the chassis, respectively, (q) each of the first connection portion and the second connection portion may comprise a plurality of discontinuous portions positioned around the plenum chamber and the chassis, respectively, and the plurality of discontinuous portions of each of the first connection portion and the second connection portion may be positioned at corresponding positions around the plenum chamber and the chassis, respectively, to connect to one another, (r) the plurality of discontinuous portions of each of the first connection portion and the second connection portion may be positioned asymmetrically around the plenum chamber and the chassis, respectively, (s) the plurality of discontinuous portions of each of the first connection portion and the second connection portion may be positioned symmetrically around the plenum chamber and the chassis, respectively, (t) the sealing lip, in an undeformed state, may have a dimension extending from the plenum chamber or the chassis in a direction that is orthogonal to a surface to which the sealing lip is attached, and the dimension may be greater than a combined thickness of the first connection portion and the second connection portion, (u) the chassis may include the vent structure, (v) an elbow may be configured to rotatably connect to the chassis at the inlet port at a first end and to an air circuit at a second end to direct the flow of air into the plenum chamber, (w) the elbow may include the vent structure, (x) the sealing lip may be constructed from an elastomeric material, (y) the sealing lip may be constructed from silicone, (z) the plenum chamber and the seal-forming structure may be formed in one piece from a homogeneous material, (aa) the plenum chamber and the seal-forming structure may be formed in one piece from a textile, (bb) the plenum chamber and the seal-forming structure may be formed in one piece from silicone, (cc) the sealing lip may be integrally molded to the plenum chamber, (dd) the sealing lip may be integrally molded to the chassis, (ee) the sealing lip may be radially continuous, (ff) two inlet ports may be positioned on the chassis, the positioning and stabilising structure may comprise a pair of conduits, each configured to connect to a corresponding one of the inlet ports to direct the flow of air into the plenum chamber, (gg) the chassis may comprise a pair of tie attachment structures and the positioning and stabilising structure may comprise a pair of ties, each of the ties being configured to attach to a corresponding one of the tie attachment structures to connect the positioning and stabilising structure to the chassis, (hh) the first connection portion may be joined to the plenum chamber with adhesive, (ii) the plenum chamber may be overmolded to the first connection portion, (jj) the first connection portion may be molded into the plenum chamber, (kk) the sealing lip may be positioned such that an increase in pressure within the plenum chamber increases a sealing force of the sealing lip between the plenum chamber and the chassis, (11) the second connection portion may be positioned on the chassis with a gap between a peripheral edge of the chassis and an outer peripheral edge of the second connection portion, (mm) the seal-forming structure may be configured so as not to enter the patient's mouth in use, (nn) the seal-forming structure may be configured so that the seal-forming structure does not extend internally of the patient's airways in use, (oo) the seal-forming structure may be configured so as not extend to below a mental protuberance region of the patient in use, (pp) the seal-forming structure and the plenum chamber may be configured so as not to cover the patient's eyes in use, (qq) the seal-forming structure may comprise a nasal hole configured to direct the flow of air into the patient's nares during use, (rr) the seal-forming structure may comprise an oral hole configured to direct the flow of air into the patient's mouth during use, (ss) the sealing lip may be cantilevered from the chassis or the plenum chamber, and/or (tt) the sealing lip may be fixed to the plenum chamber or the chassis at a first end and is free at a second end and the sealing lip is curved between the first end and the second end.

Another aspect of one form of the present technology is a patient interface that is moulded or otherwise constructed with a perimeter shape which is complementary to that of an intended wearer.

An aspect of one form of the present technology is a method of manufacturing apparatus.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a portable RPT device that may be carried by a person, e.g., around the home of the person.

An aspect of one form of the present technology is a patient interface that may be washed in a home of a patient, e.g., in soapy water, without requiring specialised cleaning equipment. An aspect of one form of the present technology is a humidifier tank that may be washed in a home of a patient, e.g., in soapy water, without requiring specialised cleaning equipment.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

### 3 BRIEF DESCRIPTION OF THE DRAWINGS

The present technology is illustrated by way of example, and not by way of limitation, in the figures of the accompanying drawings, in which like reference numerals refer to similar elements including:

### 3.1 RESPIRATORY THERAPY SYSTEMS

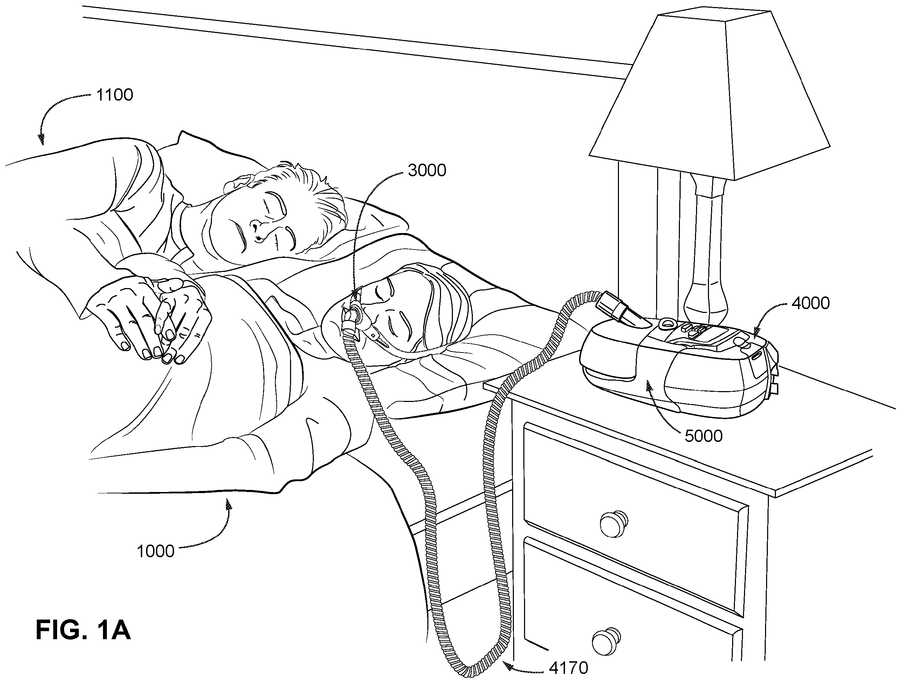

Fig. 1A shows a system including a patient 1000 wearing a patient interface 3000, in the form of nasal pillows, receiving a supply of air at positive pressure from an RPT device 4000. Air from the RPT device 4000 is humidified in a humidifier 5000, and passes along an air circuit 4170 to the patient 1000. A bed partner 1100 is also shown. The patient is sleeping in a supine sleeping position.

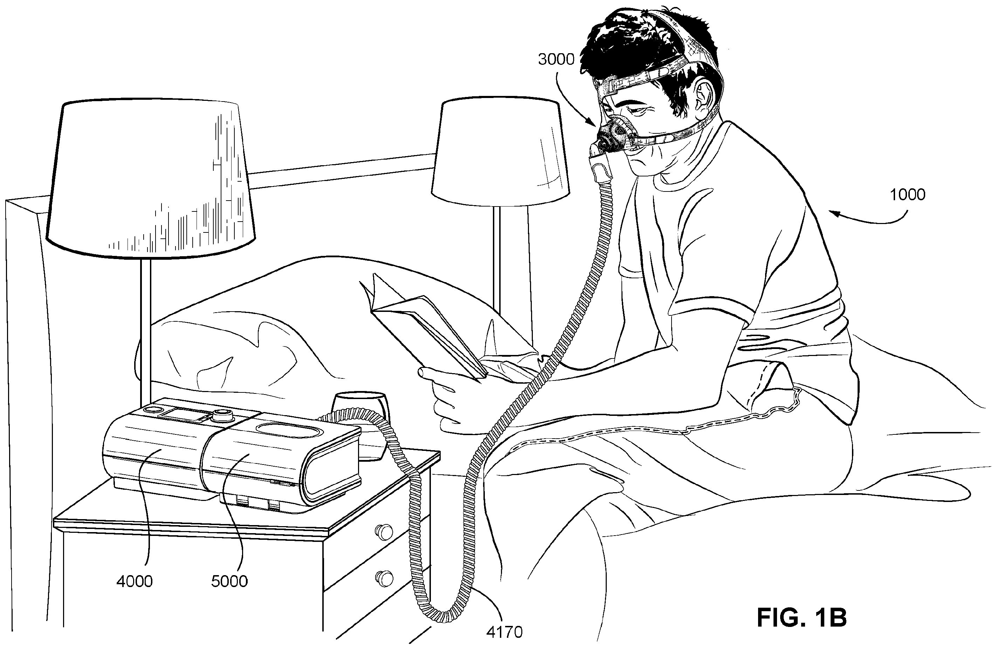

Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.

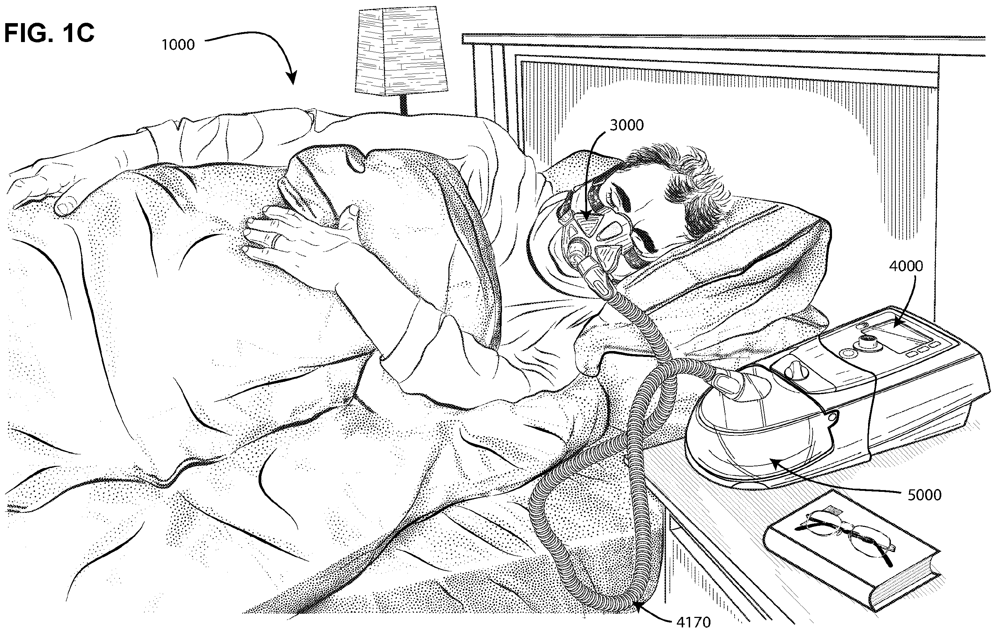

Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

### 3.2 RESPIRATORY SYSTEM AND FACIAL ANATOMY

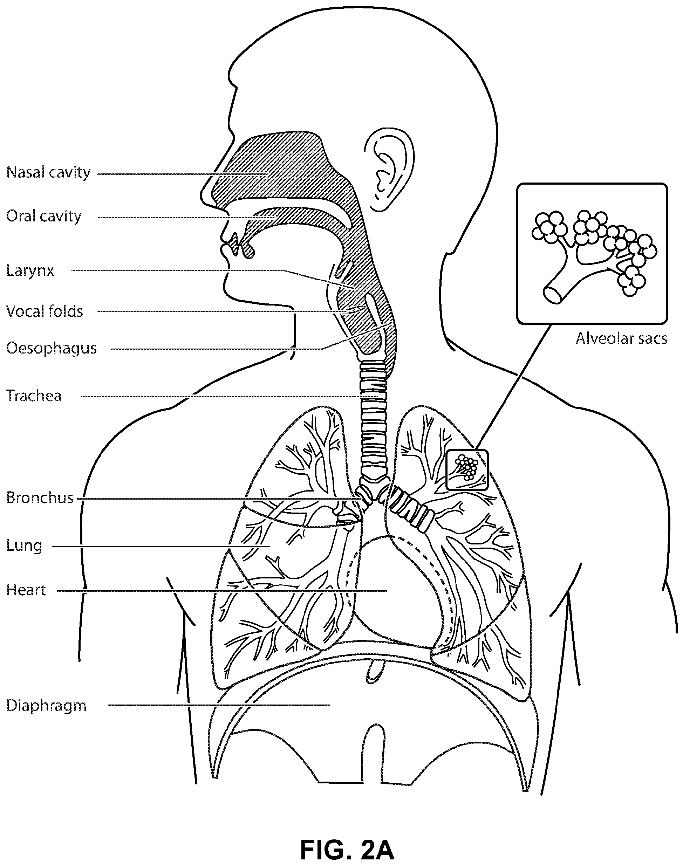

Fig. 2A shows an overview of a human respiratory system including the nasal and oral cavities, the larynx, vocal folds, oesophagus, trachea, bronchus, lung, alveolar sacs, heart and diaphragm.

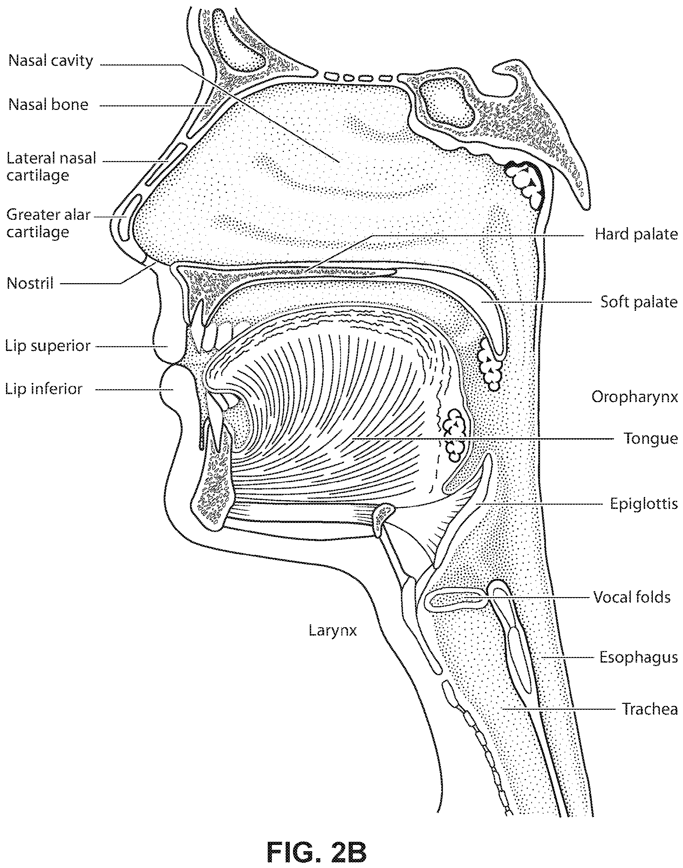

Fig. 2B shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.

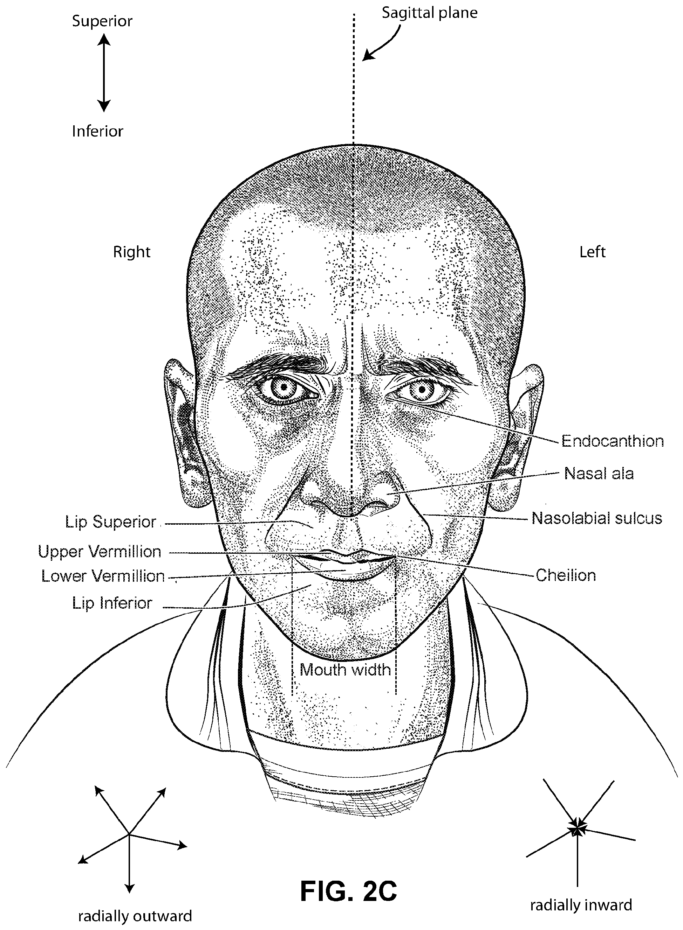

Fig. 2C is a front view of a face with several features of surface anatomy identified including the lip superior, upper vermilion, lower vermilion, lip inferior, mouth width, endocanthion, a nasal ala, nasolabial sulcus and cheilion. Also indicated are the directions superior, inferior, radially inward and radially outward.

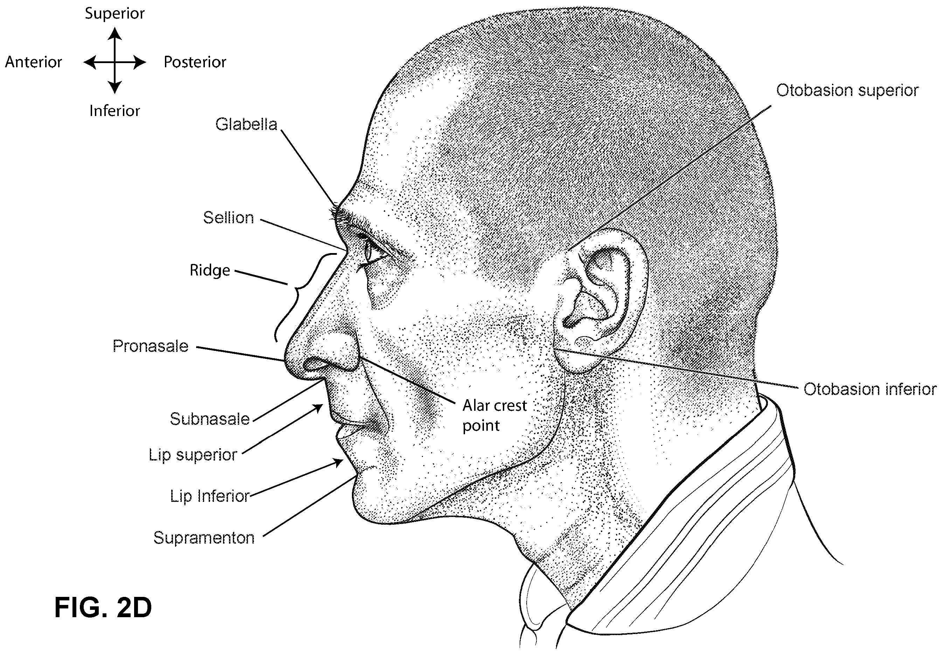

Fig. 2D is a side view of a head with several features of surface anatomy identified including glabella, sellion, pronasale, subnasale, lip superior, lip inferior, supramenton, nasal ridge, alar crest point, otobasion superior and otobasion inferior. Also indicated are the directions superior & inferior, and anterior & posterior.

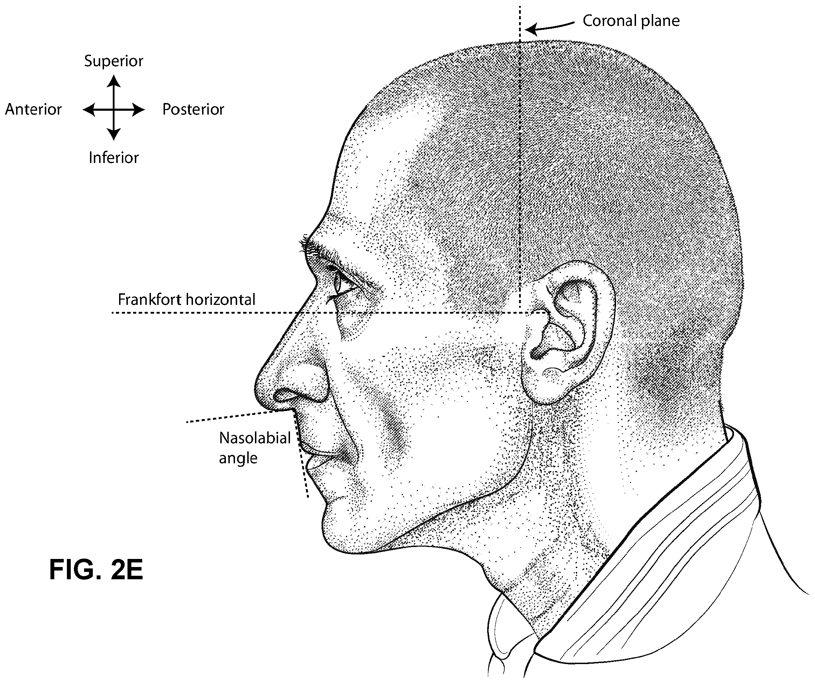

Fig. 2E is a further side view of a head. The approximate locations of the Frankfort horizontal and nasolabial angle are indicated. The coronal plane is also indicated.

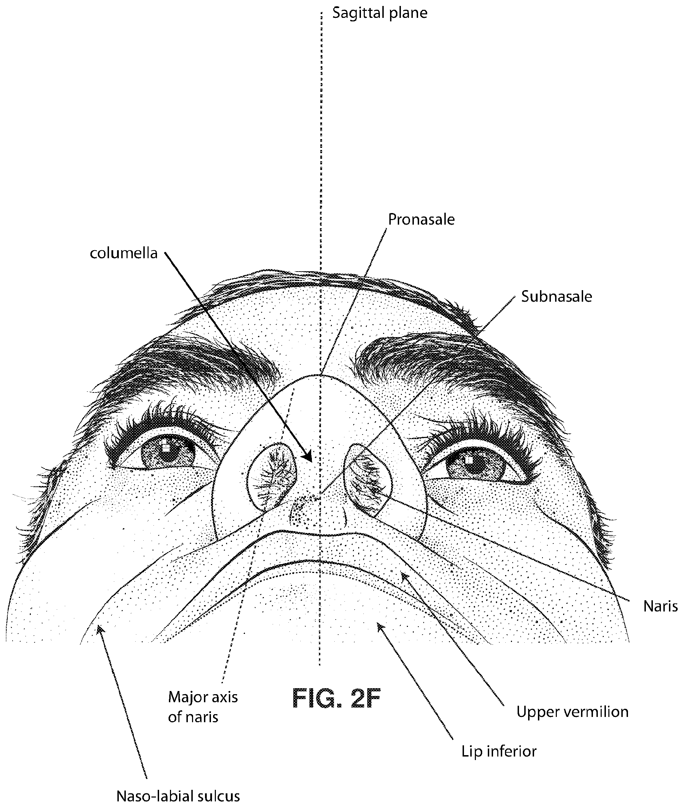

Fig. 2F shows a base view of a nose with several features identified including naso-labial sulcus, lip inferior, upper Vermilion, naris, subnasale, columella, pronasale, the major axis of a naris and the midsagittal plane.

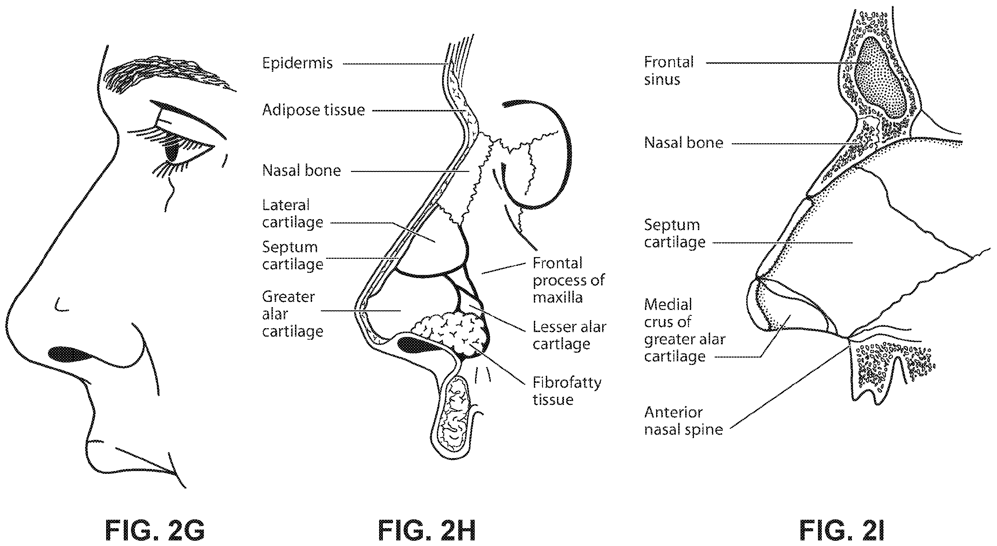

Fig. 2G shows a side view of the superficial features of a nose.
Fig. 2H shows subcutaneal structures of the nose, including lateral cartilage, septum cartilage, greater alar cartilage, lesser alar cartilage, sesamoid cartilage, nasal bone, epidermis, adipose tissue, frontal process of the maxilla and fibrofatty tissue.
Fig. 2I shows a medial dissection of a nose, approximately several millimeters from the midsagittal plane, amongst other things showing the septum cartilage and medial crus of greater alar cartilage.

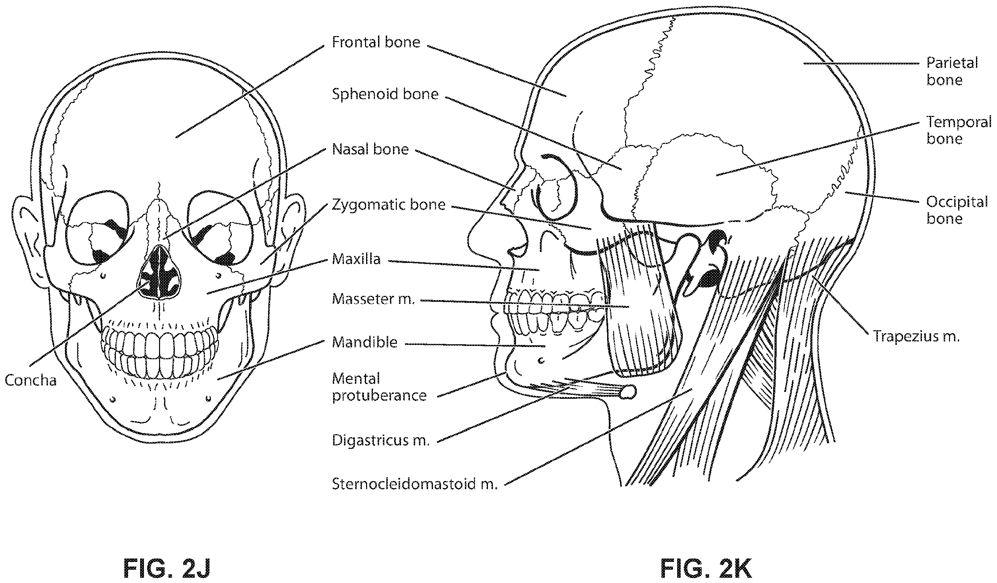

Fig. 2J shows a front view of the bones of a skull including the frontal, nasal and zygomatic bones. Nasal concha are indicated, as are the maxilla, and mandible.
Fig. 2K shows a lateral view of a skull with the outline of the surface of a head, as well as several muscles. The following bones are shown: frontal, sphenoid, nasal, zygomatic, maxilla, mandible, parietal, temporal and occipital. The mental protuberance is indicated. The following muscles are shown: digastricus, masseter, sternocleidomastoid and trapezius.

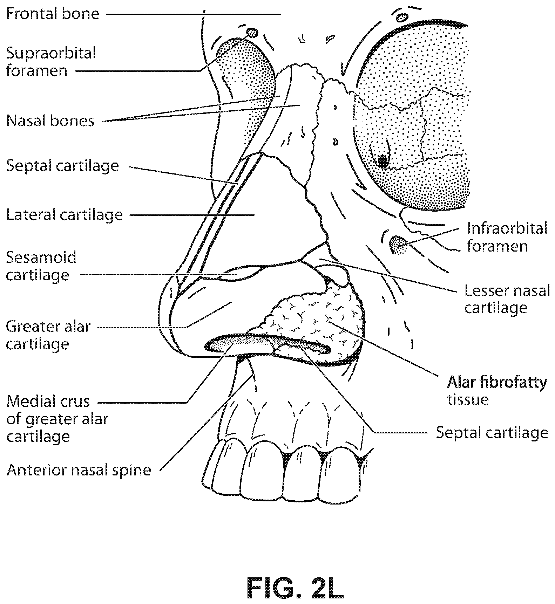

Fig. 2L shows an anterolateral view of a nose.

### 3.3 PATIENT INTERFACE

Fig. 3A shows a patient interface in the form of a nasal mask in accordance with one form of the present technology.
Fig. 3B shows a schematic of a cross-section through a structure at a point. An outward normal at the point is indicated. The curvature at the point has a positive sign, and a relatively large magnitude when compared to the magnitude of the curvature shown in Fig. 3C.
Fig. 3C shows a schematic of a cross-section through a structure at a point. An outward normal at the point is indicated. The curvature at the point has a positive sign, and a relatively small magnitude when compared to the magnitude of the curvature shown in Fig. 3B.
Fig. 3D shows a schematic of a cross-section through a structure at a point. An outward normal at the point is indicated. The curvature at the point has a value of zero.
Fig. 3E shows a schematic of a cross-section through a structure at a point. An outward normal at the point is indicated. The curvature at the point has a negative sign, and a relatively small magnitude when compared to the magnitude of the curvature shown in Fig. 3F.
Fig. 3F shows a schematic of a cross-section through a structure at a point. An outward normal at the point is indicated. The curvature at the point has a negative sign, and a relatively large magnitude when compared to the magnitude of the curvature shown in Fig. 3E.
Fig. 3G shows a cushion for a mask that includes two pillows. An exterior surface of the cushion is indicated. An edge of the surface is indicated. Dome and saddle regions are indicated.
Fig. 3H shows a cushion for a mask. An exterior surface of the cushion is indicated. An edge of the surface is indicated. A path on the surface between points A and B is indicated. A straight line distance between A and B is indicated. Two saddle regions and a dome region are indicated.
Fig. 3I shows the surface of a structure, with a one dimensional hole in the surface. The illustrated plane curve forms the boundary of a one dimensional hole.
Fig. 3J shows a cross-section through the structure of Fig.3I. The illustrated surface bounds a two dimensional hole in the structure of Fig. 3I.
Fig. 3K shows a perspective view of the structure of Fig. 3I, including the two dimensional hole and the one dimensional hole. Also shown is the surface that bounds a two dimensional hole in the structure of Fig. 3I.
Fig. 3L shows a mask having an inflatable bladder as a cushion.
Fig. 3M shows a cross-section through the mask of Fig. 3L, and shows the interior surface of the bladder. The interior surface bounds the two dimensional hole in the mask.
Fig. 3N shows a further cross-section through the mask of Fig. 3L. The interior surface is also indicated.
Fig. 3O illustrates a left-hand rule.
Fig. 3P illustrates a right-hand rule.
Fig. 3Q shows a left ear, including the left ear helix.
Fig. 3R shows a right ear, including the right ear helix.
Fig. 3S shows a right-hand helix.
Fig. 3T shows a view of a mask, including the sign of the torsion of the space curve defined by the edge of the sealing membrane in different regions of the mask.
Fig. 3U shows a view of a plenum chamber 3200 showing a sagittal plane and a mid-contact plane.
Fig. 3V shows a view of a posterior of the plenum chamber of Fig. 3U. The direction of the view is normal to the mid-contact plane. The sagittal plane in Fig. 3V bisects the plenum chamber into left-hand and right-hand sides.

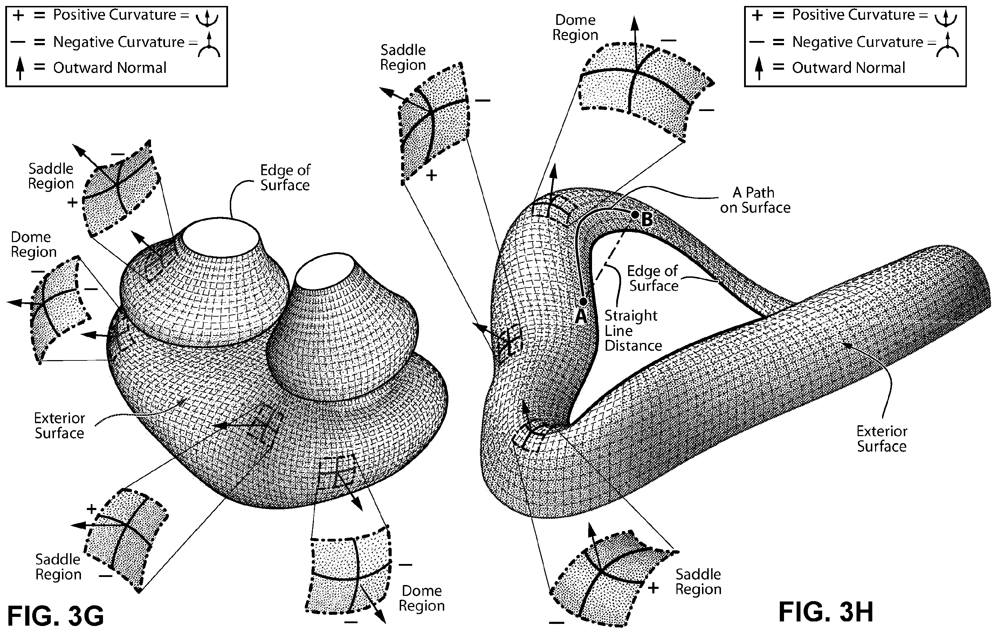

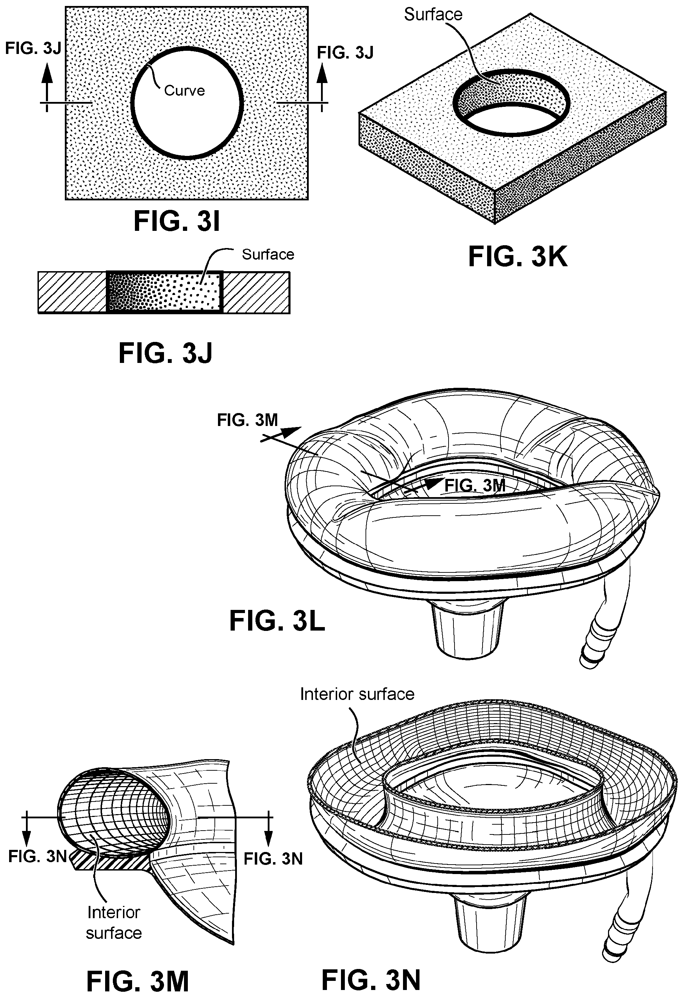

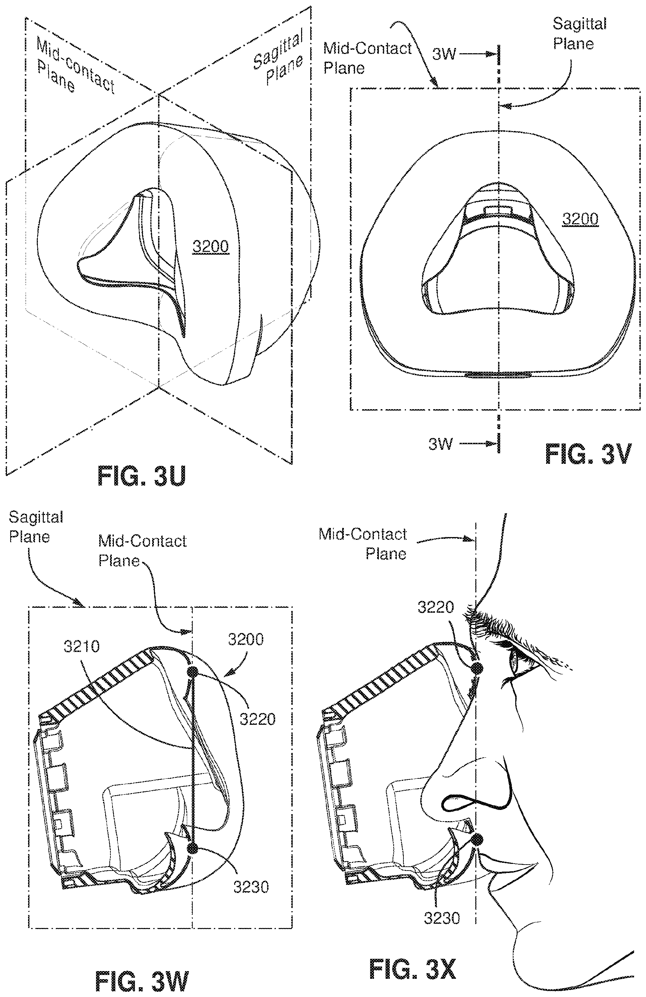

Fig. 3W shows a cross-section through the plenum chamber of Fig. 3V, the cross-section being taken at the sagittal plane shown in Fig. 3V. A 'mid-contact' plane is shown. The mid-contact plane is perpendicular to the sagittal plane. The orientation of the mid-contact plane corresponds to the orientation of a chord 3210 which lies on the sagittal plane and just touches the cushion of the plenum chamber at two points on the sagittal plane: a superior point 3220 and an inferior point 3230. Depending on the geometry of the cushion in this region, the mid-contact plane may be a tangent at both the superior and inferior points.
Fig. 3X shows the plenum chamber 3200 of Fig. 3U in position for use on a face. The sagittal plane of the plenum chamber 3200 generally coincides with the midsagittal plane of the face when the plenum chamber is in position for use. The mid-contact plane corresponds generally to the 'plane of the face' when the plenum chamber is in position for use. In Fig. 3X the plenum chamber 3200 is that of a nasal mask, and the superior point 3220 sits approximately on the sellion, while the inferior point 3230 sits on the lip superior.

### 3.4 RPT DEVICE

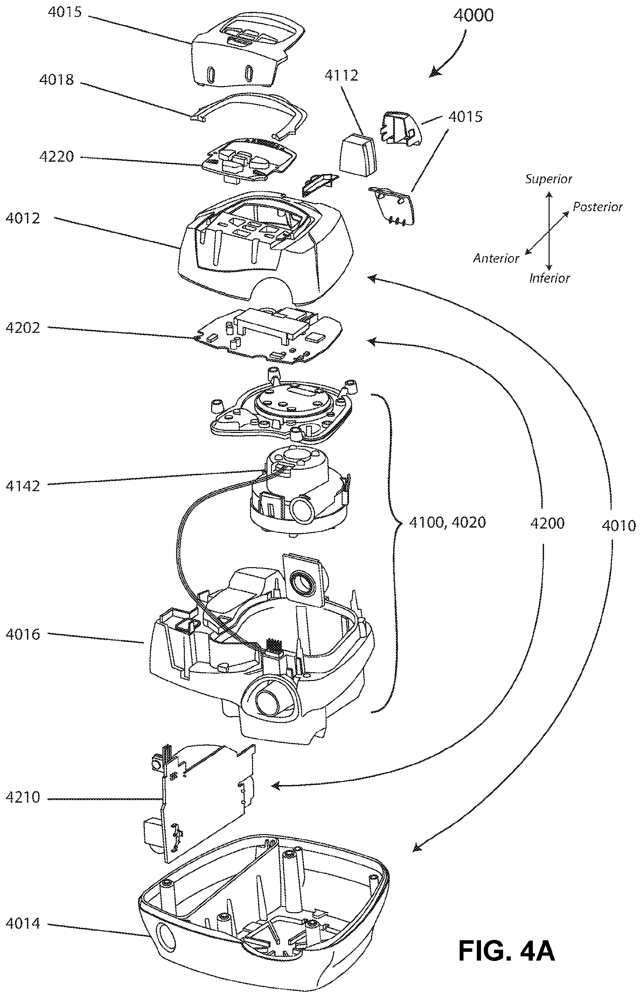

Fig. 4A shows an RPT device in accordance with one form of the present technology.

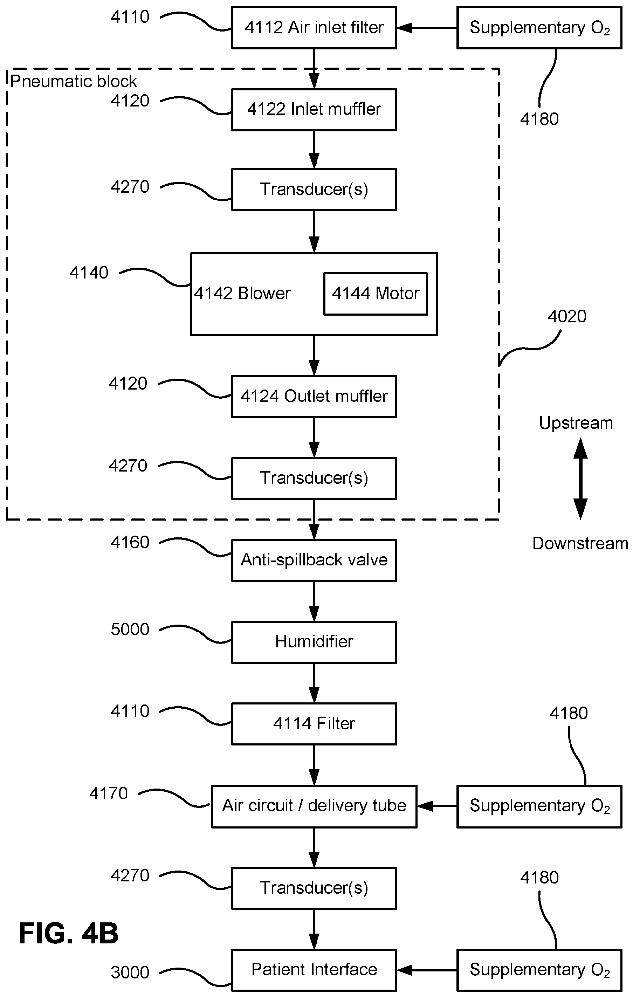

Fig. 4B is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

### 3.5 HUMIDIFIER

Fig. 5A shows an isometric view of a humidifier in accordance with one form of the present technology.
Fig. 5B shows an isometric view of a humidifier in accordance with one form of the present technology, showing a humidifier reservoir 5110 removed from the humidifier reservoir dock 5130.

### 3.6 BREATHING WAVEFORMS

Fig. 6 shows a model typical breath waveform of a person while sleeping.

### 3.7 PATIENT INTERFACE WITH DETACHABLE, SEALED CHASSIS

Fig. 7A shows a front view of a seal-forming structure and a plenum chamber according to an example of the present technology.
Fig. 7B shows a front view of a chassis according to an example of the present technology.
Fig. 8 shows a front view of a chassis attached to a seal-forming structure and a plenum chamber according to an example of the present technology.
Fig. 9 shows a front view of a chassis attached to a seal-forming structure and a plenum chamber according to an example of the present technology.
Fig. 10A shows a top view of a chassis detached from a seal-forming structure and a plenum chamber according to an example of the present technology.
Fig. 10B shows a detailed top view of a chassis detached from a seal-forming structure and a plenum chamber according to an example of the present technology.
Fig. 10C shows a detailed top view of a chassis attached to a seal-forming structure and a plenum chamber according to an example of the present technology.
Fig. 11A shows a top view of a chassis detached from a seal-forming structure and a plenum chamber according to an example of the present technology.
Fig. 11B shows a detailed top view of a chassis detached from a seal-forming structure and a plenum chamber according to an example of the present technology.
Fig. 11C shows a detailed top view of a chassis attached to a seal-forming structure and a plenum chamber according to an example of the present technology.
Fig. 12 shows a front view of a seal-forming structure and a plenum chamber according to another example of the present technology.
Fig. 13A shows a front view of a seal-forming structure and a plenum chamber according to an example of the present technology.
Fig. 13B shows a rear view of a chassis according to an example of the present technology.

### 4 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

### 4.1 THERAPY

In one form, the present technology comprises a method for treating a respiratory disorder comprising applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

### 4.2 RESPIRATORY THERAPY SYSTEMS

In one form, the present technology comprises a respiratory therapy system for treating a respiratory disorder. The respiratory therapy system may comprise an RPT device 4000 for supplying a flow of air to the patient 1000 via an air circuit 4170 and a patient interface 3000.

### 4.3 PATIENT INTERFACE

A non-invasive patient interface 3000 in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy.

If a patient interface is unable to comfortably deliver a minimum level of positive pressure to the airways, the patient interface may be unsuitable for respiratory pressure therapy.

The patient interface 3000 in accordance with one form of the present technology is constructed and arranged to be able to provide a supply of air at a positive pressure of at least 6 cmH2O with respect to ambient.

The patient interface 3000 in accordance with one form of the present technology is constructed and arranged to be able to provide a supply of air at a positive pressure of at least 10 cmH2O with respect to ambient.

The patient interface 3000 in accordance with one form of the present technology is constructed and arranged to be able to provide a supply of air at a positive pressure of at least 20 cmH2O with respect to ambient.

### 4.3.1 Seal-forming structure

In one form of the present technology, a seal-forming structure 3100 provides a target seal-forming region, and may additionally provide a cushioning function. The target seal-forming region is a region on the seal-forming structure 3100 where sealing may occur. The region where sealing actually occurs- the actual sealing surface- may change within a given treatment session, from day to day, and from patient to patient, depending on a range of factors including for example, where the patient interface was placed on the face, tension in the positioning and stabilising structure and the shape of a patient's face.

In one form the target seal-forming region is located on an outside surface of the seal-forming structure 3100.

In certain forms of the present technology, the seal-forming structure 3100 is constructed from a biocompatible material, e.g. silicone rubber.

A seal-forming structure 3100 in accordance with the present technology may be constructed from a soft, flexible, resilient material such as silicone.

In certain forms of the present technology, a system is provided comprising more than one a seal-forming structure 3100, each being configured to correspond to a different size and/or shape range. For example the system may comprise one form of a seal-forming structure 3100 suitable for a large sized head, but not a small sized head and another suitable for a small sized head, but not a large sized head.

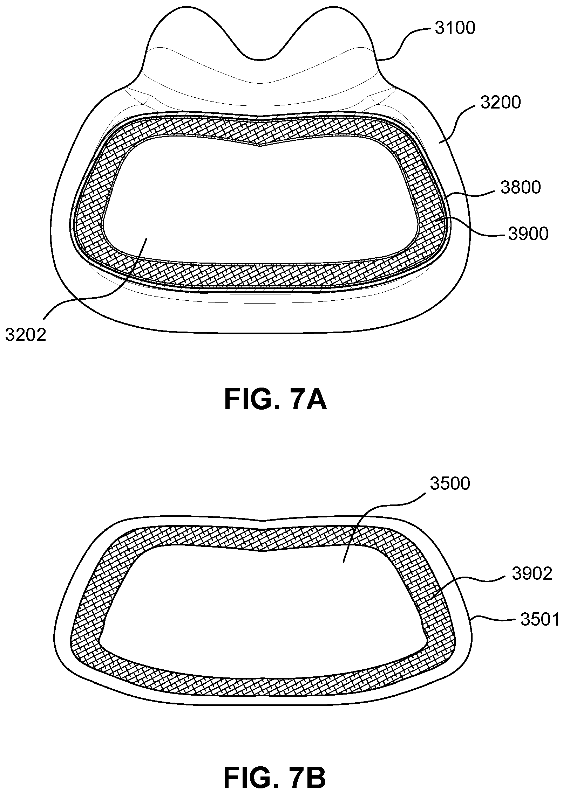

In examples of the present technology, such as those shown in Figs. 7A-13B, the seal-forming structure 3100 may be configured so as not to enter the patient's mouth in use. Also, the seal-forming structure 3100 may be configured so as not to extend internally of the patient's airways in use. Also, the seal-forming structure 3100 may be configured so as not extend to below a mental protuberance region of the patient in use. Also, the seal-forming structure 3100 and the plenum chamber 3200 may be configured so as not to cover the patient's eyes in use.

In examples of the present technology, such as those shown in Figs. 7A-13B, the seal-forming structure 3100 may include one or more nasal holes 3101 configured to direct the flow of air into the patient's nares during use. Also, the seal-forming structure 3100 may include an oral hole 3102 configured to direct the flow of air into the patient's mouth during use.

### 4.3.1.1 Sealing mechanisms

In one form, the seal-forming structure includes a sealing flange utilizing a pressure assisted sealing mechanism. In use, the sealing flange can readily respond to a system positive pressure in the interior of the plenum chamber 3200 acting on its underside to urge it into tight sealing engagement with the face. The pressure assisted mechanism may act in conjunction with elastic tension in the positioning and stabilising structure.

In one form, the seal-forming structure 3100 comprises a sealing flange and a support flange. The sealing flange comprises a relatively thin member with a thickness of less than about 1mm, for example about 0.25mm to about 0.45mm, which extends around the perimeter of the plenum chamber 3200. Support flange may be relatively thicker than the sealing flange. The support flange is disposed between the sealing flange and the marginal edge of the plenum chamber 3200, and extends at least part of the way around the perimeter. The support flange is or includes a springlike element and functions to support the sealing flange from buckling in use.

In one form, the seal-forming structure may comprise a compression sealing portion or a gasket sealing portion. In use the compression sealing portion, or the gasket sealing portion is constructed and arranged to be in compression, e.g. as a result of elastic tension in the positioning and stabilising structure.

In one form, the seal-forming structure comprises a tension portion. In use, the tension portion is held in tension, e.g. by adjacent regions of the sealing flange.

In one form, the seal-forming structure comprises a region having a tacky or adhesive surface.

In certain forms of the present technology, a seal-forming structure may comprise one or more of a pressure-assisted sealing flange, a compression sealing portion, a gasket sealing portion, a tension portion, and a portion having a tacky or adhesive surface.

### 4.3.1.2 Nose bridge or nose ridge region

In one form, the non-invasive patient interface 3000 comprises a seal-forming structure that forms a seal in use on a nose bridge region or on a nose-ridge region of the patient's face.

In one form, the seal-forming structure includes a saddle-shaped region constructed to form a seal in use on a nose bridge region or on a nose-ridge region of the patient's face.

### 4.3.1.3 Upper lip region

In one form, the non-invasive patient interface 3000 comprises a seal-forming structure that forms a seal in use on an upper lip region (that is, the lip superior) of the patient's face.

In one form, the seal-forming structure includes a saddle-shaped region constructed to form a seal in use on an upper lip region of the patient's face.

### 4.3.1.4 Chin-region

In one form the non-invasive patient interface 3000 comprises a seal-forming structure that forms a seal in use on a chin-region of the patient's face.

In one form, the seal-forming structure includes a saddle-shaped region constructed to form a seal in use on a chin-region of the patient's face.

### 4.3.1.5 Forehead region

In one form, the seal-forming structure that forms a seal in use on a forehead region of the patient's face. In such a form, the plenum chamber may cover the eyes in use.

### 4.3.1.6 Nasal pillows

In one form the seal-forming structure of the non-invasive patient interface 3000 comprises a pair of nasal puffs, or nasal pillows, each nasal puff or nasal pillow being constructed and arranged to form a seal with a respective naris of the nose of a patient.

Nasal pillows in accordance with an aspect of the present technology include: a frusto-cone, at least a portion of which forms a seal on an underside of the patient's nose, a stalk, a flexible region on the underside of the frusto-cone and connecting the frusto-cone to the stalk. In addition, the structure to which the nasal pillow of the present technology is connected includes a flexible region adjacent the base of the stalk. The flexible regions can act in concert to facilitate a universal joint structure that is accommodating of relative movement both displacement and angular of the frusto-cone and the structure to which the nasal pillow is connected. For example, the frusto-cone may be axially displaced towards the structure to which the stalk is connected.

### 4.3.2 Plenum chamber

The plenum chamber 3200 has a perimeter that is shaped to be complementary to the surface contour of the face of an average person in the region where a seal will form in use. In use, a marginal edge of the plenum chamber 3200 is positioned in close proximity to an adjacent surface of the face. Actual contact with the face is provided by the seal-forming structure 3100. The seal-forming structure 3100 may extend in use about the entire perimeter of the plenum chamber 3200. In some forms, the plenum chamber 3200 and the seal-forming structure 3100 are formed from a single homogeneous piece of material. In some forms, the plenum chamber 3200 and the seal-forming structure 3100 form a unitary structure, e.g., a cushion assembly, and each may be constructed from a respective material having at least one different property or the plenum chamber 3200 and the seal-forming structure 3100 may be formed from a single homogeneous piece of material.

In certain forms of the present technology, the plenum chamber 3200 does not cover the eyes of the patient in use. In other words, the eyes are outside the pressurised volume defined by the plenum chamber. Such forms tend to be less obtrusive and / or more comfortable for the wearer, which can improve compliance with therapy.

In certain forms of the present technology, the plenum chamber 3200 is constructed from a transparent material, e.g. a transparent polycarbonate. The use of a transparent material can reduce the obtrusiveness of the patient interface, and help improve compliance with therapy. The use of a transparent material can aid a clinician to observe how the patient interface is located and functioning.

In certain forms of the present technology, the plenum chamber 3200 is constructed from a translucent material. The use of a translucent material can reduce the obtrusiveness of the patient interface, and help improve compliance with therapy.

### 4.3.3 Positioning and stabilising structure

The seal-forming structure 3100 of the patient interface 3000 of the present technology may be held in sealing position in use by the positioning and stabilising structure 3300.

In one form the positioning and stabilising structure 3300 provides a retention force at least sufficient to overcome the effect of the positive pressure in the plenum chamber 3200 to lift off the face.

In one form the positioning and stabilising structure 3300 provides a retention force to overcome the effect of the gravitational force on the patient interface 3000.

In one form the positioning and stabilising structure 3300 provides a retention force as a safety margin to overcome the potential effect of disrupting forces on the patient interface 3000, such as from tube drag, or accidental interference with the patient interface.

In one form of the present technology, a positioning and stabilising structure 3300 is provided that is configured in a manner consistent with being worn by a patient while sleeping. In one example the positioning and stabilising structure 3300 has a low profile, or cross-sectional thickness, to reduce the perceived or actual bulk of the apparatus. In one example, the positioning and stabilising structure 3300 comprises at least one strap having a rectangular cross-section. In one example the positioning and stabilising structure 3300 comprises at least one flat strap.

In one form of the present technology, a positioning and stabilising structure 3300 is provided that is configured so as not to be too large and bulky to prevent the patient from lying in a supine sleeping position with a back region of the patient's head on a pillow.

In one form of the present technology, a positioning and stabilising structure 3300 is provided that is configured so as not to be too large and bulky to prevent the patient from lying in a side sleeping position with a side region of the patient's head on a pillow.

In one form of the present technology, a positioning and stabilising structure 3300 is provided with a decoupling portion located between an anterior portion of the positioning and stabilising structure 3300, and a posterior portion of the positioning and stabilising structure 3300. The decoupling portion does not resist compression and may be, e.g. a flexible or floppy strap. The decoupling portion is constructed and arranged so that when the patient lies with their head on a pillow, the presence of the decoupling portion prevents a force on the posterior portion from being transmitted along the positioning and stabilising structure 3300 and disrupting the seal.

In one form of the present technology, a positioning and stabilising structure 3300 comprises a strap constructed from a laminate of a fabric patient-contacting layer, a foam inner layer and a fabric outer layer. In one form, the foam is porous to allow moisture, (e.g., sweat), to pass through the strap. In one form, the fabric outer layer comprises loop material to engage with a hook material portion.

In certain forms of the present technology, a positioning and stabilising structure 3300 comprises a strap that is extensible, e.g. resiliently extensible. For example the strap may be configured in use to be in tension, and to direct a force to draw a seal-forming structure into sealing contact with a portion of a patient's face. In an example the strap may be configured as a tie.

In one form of the present technology, the positioning and stabilising structure comprises a first tie, the first tie being constructed and arranged so that in use at least a portion of an inferior edge thereof passes superior to an otobasion superior of the patient's head and overlays a portion of a parietal bone without overlaying the occipital bone.

In one form of the present technology suitable for a nasal-only mask or for a full-face mask, the positioning and stabilising structure includes a second tie, the second tie being constructed and arranged so that in use at least a portion of a superior edge thereof passes inferior to an otobasion inferior of the patient's head and overlays or lies inferior to the occipital bone of the patient's head.

In one form of the present technology suitable for a nasal-only mask or for a full-face mask, the positioning and stabilising structure includes a third tie that is constructed and arranged to interconnect the first tie and the second tie to reduce a tendency of the first tie and the second tie to move apart from one another.

In certain forms of the present technology, a positioning and stabilising structure 3300 comprises a strap that is bendable and e.g. non-rigid. An advantage of this aspect is that the strap is more comfortable for a patient to lie upon while the patient is sleeping.

In certain forms of the present technology, a positioning and stabilising structure 3300 comprises a strap constructed to be breathable to allow moisture vapour to be transmitted through the strap,

In certain forms of the present technology, a system is provided comprising more than one positioning and stabilizing structure 3300, each being configured to provide a retaining force to correspond to a different size and/or shape range. For example the system may comprise one form of positioning and stabilizing structure 3300 suitable for a large sized head, but not a small sized head, and another. suitable for a small sized head, but not a large sized head.

### 4.3.4 Vent

In one form, the patient interface 3000 includes a vent 3400 constructed and arranged to allow for the washout of exhaled gases, e.g. carbon dioxide.

In certain forms the vent 3400 is configured to allow a continuous vent flow from an interior of the plenum chamber 3200 to ambient whilst the pressure within the plenum chamber is positive with respect to ambient. The vent 3400 is configured such that the vent flow rate has a magnitude sufficient to reduce rebreathing of exhaled CO2 by the patient while maintaining the therapeutic pressure in the plenum chamber in use.

One form of vent 3400 in accordance with the present technology comprises a plurality of holes, for example, about 20 to about 80 holes, or about 40 to about 60 holes, or about 45 to about 55 holes.

The vent 3400 may be located in the plenum chamber 3200. Alternatively, the vent 3400 is located in a decoupling structure, e.g., a swivel.

### 4.3.5 Decoupling structure(s)

In one form the patient interface 3000 includes at least one decoupling structure, for example, a swivel or a ball and socket.

### 4.3.6 Connection port

Connection port 3600 allows for connection to the air circuit 4170.

### 4.3.7 Forehead support

In one form, the patient interface 3000 includes a forehead support 3700.

### 4.3.8 Anti-asphyxia valve

In one form, the patient interface 3000 includes an anti-asphyxia valve.

### 4.3.9 Ports

In one form of the present technology, a patient interface 3000 includes one or more ports that allow access to the volume within the plenum chamber 3200. In one form this allows a clinician to supply supplementary oxygen. In one form, this allows for the direct measurement of a property of gases within the plenum chamber 3200, such as the pressure.

### 4.3.10 Detachable, sealed chassis

Figs. 7A-13B show examples of a patient interface 3000 with a chassis 3500 that may be detachable from the plenum chamber 3200 and in which there may be a seal between the chassis 3500 and the plenum chamber 3200 when those components are attached. In the examples described below, the plenum chamber 3200 may include a first connection portion 3900 and the chassis 3500 may include a second connection portion 3902, the first connection portion 3900 and the second connection portion 3902 being releasably connectable. The chassis 3500, when the first connection portion 3900 and the second connection portion 3902 are connected, may cover a plenum chamber opening 3202 on an anterior side of the plenum chamber 3200. Also in the examples described below, a sealing lip 3800 may seal between the plenum chamber 3200 and the chassis 3500 when connected to allow a pressure greater than ambient to be maintained within the plenum chamber 3200 during therapy.

### 4.3.10.1 Chassis

In these examples, the chassis 3500 may be constructed from a flexible material. The flexible material may be textile, which in further examples may be air-impermeable, or silicone. If the flexible material is an air-impermeable textile, the textile may be laminated or impregnated with a polymeric material.

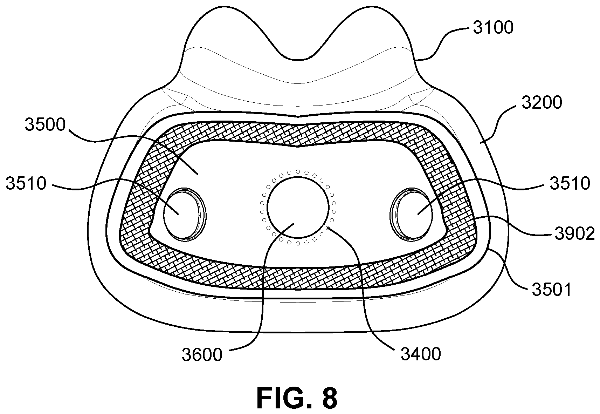

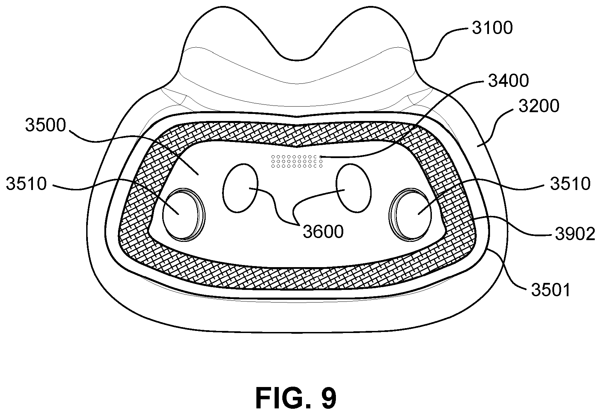

The chassis 3500 may include a connection or inlet port 3600 for removable connection to an air circuit 4170 directly or via an elbow, as in Fig. 8. If an elbow is attached to the chassis 3500 to connect the air circuit 4170, the elbow may be rotatable. Alternatively, Fig. 9 shows an example in which one connection port 3600 is positioned on each lateral side of the chassis 3500. In this example, the positioning and stabilising structure 3300 may include a pair of conduits, each positioned on a corresponding lateral side of the patient's head in use, and each of the conduits may be connected to a corresponding one of the connection ports 3600 to direct the flow of air into the plenum chamber 3200. If the chassis 3500 is constructed from a relatively flexible material, such as textile or silicone, the chassis 3500 may include a relatively rigid structure at the connection port 3600 to facilitate a secure connection with the elbow, air circuit 4170, or conduits.

The chassis 3500 may include the vent structure 3400, as shown in Figs. 8 and 9. Alternatively, the elbow attached to the inlet port 3600 may include the vent structure 3400.

The chassis 3500 may also include one or more tie attachment structures 3510 to which clips may be releasably attached, and the clips may in turn be releasably attached to respective ties of the positioning and stabilising structure 3300. Each tie attachment structure 3510 may include a magnet and each clip may include a magnet, and the poles of those magnets may be oriented to attract one another. Figs. 8 and 9 show examples of two tie attachment structures 3510 on the chassis 3500, but it should be understood that more or fewer tie attachment structures 3510 may be included depending on the chosen configuration of positioning and stabilising structure 3300.

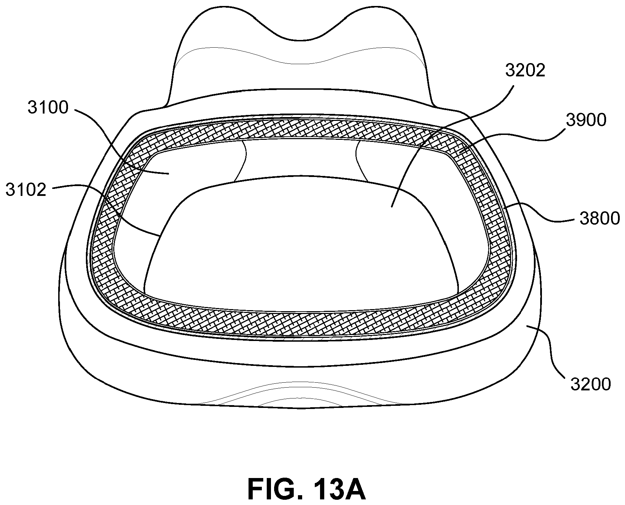

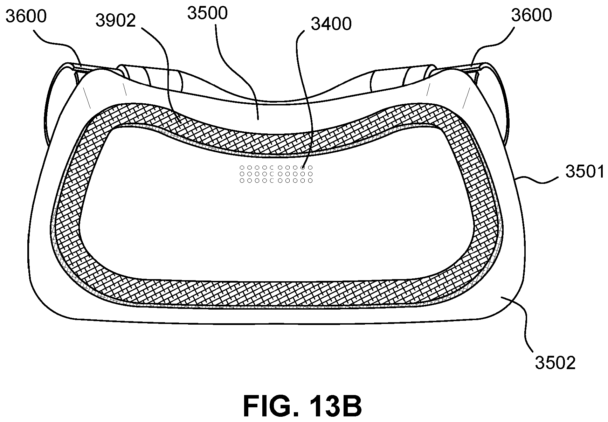

Figs. 13A and 13B show a further example in which the chassis 3500 includes an inlet port 3600 positioned on each lateral side thereof for attachment to a corresponding conduit of the positioning and stabilising structure 3300. Although not shown in these views, the chassis 3500 may also include tie attachment structures 3510 opposite the side shown in Fig. 13B, similar to Figs. 8 and 9.

### 4.3.10.2 Connection portions

The plenum chamber 3200 may include the first connection portion 3900 positioned proximal to the plenum chamber opening 3202, and the chassis 3500 may include the second connection portion 3902 for releasably connecting to the first connection portion 3900. The first connection portion 3900 may be positioned radially outward of the plenum chamber opening 3202, as shown in Fig. 7A for example. Also, Fig. 7B shows, for example, that the second connection portion 3902 may be positioned radially inward of a peripheral edge 3501 of the chassis 3500. The first connection portion 3900 and the second connection portion 3902 may each be sized and shaped to correspond to the size and shape of the plenum chamber 3200 and the chassis 3500, respectively.

In examples of the present technology, the first connection portion 3900 may be one of a hook material and a loop material and the second connection portion 3902 may be the other of the hook material and the loop material. The hook material, the loop material, or both may be constructed from a polymer (e.g., plastic), which can be disinfected frequently without significantly reducing its structural integrity.

In a further example, one of the first connection portion 3900 or the second connection portion 3902 may be a reconnectable adhesive and the other is a flat surface on the plenum chamber 3200 or the chassis 3500.

The hook material and the loop material may be of sufficient thickness and rigidity such that when the first connection portion 3900 and the second connection portion 3902 are connected, the combined connection portions are more rigid than the chassis 3500, the plenum chamber 3200, or both. The chassis 3500 and/or the plenum chamber 3200 may be constructed from a relatively flexible materials, such as textile or silicone, which may improve comfort the patient. Furthermore, in these examples, the plenum chamber 3200 and the seal-forming structure 3100 may be formed from a single piece of homogeneous material, such as textile or silicone. The increased rigidity due to connecting the connection portions 3900, 3902 may provide greater structural rigidity for the patient interface 3000 to resist deformation that may be caused by the patient's head against a pillow, tension from the positioning and stabilising structure 3300, and/or tube drag from the air circuit 4170.

Also, depending on the possible effects of tube drag from the air circuit 4170, as in the version of Fig. 8, the first connection portion 3900 and second connection portion 3902 may be widened to increase to the surface area of their connection, thereby making the connection more secure. The increased width may also increase the structural rigidity of the patient interface 3000 to resist deformation due to tube drag. In the version of Fig. 9, the width of the first connection portion 3900 and second connection portion 3902 may be comparatively less because the chassis 3500 would not experience tube drag from the air circuit 4170.

In the example of Fig. 7A, the first connection portion 3900 and the second connection portion 3902 are continuous around the plenum chamber 3200 and the chassis 3500, respectively. The first connection portion 3900 and the second connection portion 3902 may be sized and shaped complementarily so that a secure connection is formed upon engagement.

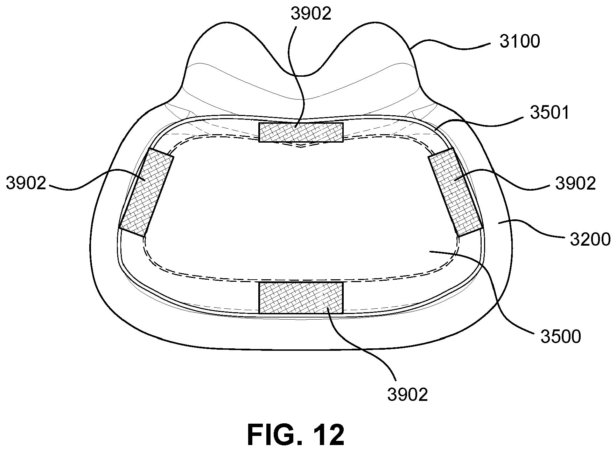

Fig. 12 shows an example in which each of the first connection portion 3900 and the second connection portion 3902 comprise a plurality of discontinuous portions of the hook material and the loop material, respectively. Those discrete portions may be positioned around the plenum chamber 3200 and the chassis 3500, respectively, at corresponding positions to connect to one another.

In a further example, one of the first connection portion 3900 and the second connection portion 3902 includes a plurality of discontinuous portions of the hook material or the loop material positioned around a corresponding one of the plenum chamber 3200 or the chassis 3500. The other of the first connection portion 3900 and the second connection portion 3902 may be a continuous piece of the other of the hook material or the loop material positioned around the plenum chamber 3200 or the chassis 3500, respectively.

In further examples, the plurality of discontinuous portions of the first connection portion 3900, the second connection portion 3902, or both, as the case may be, are positioned symmetrically or asymmetrically around the plenum chamber 3200 and the chassis 3500, respectively. Such arrangements may prohibit the chassis 3500 from being connected to the plenum chamber 3200 in an unintended orientation, which could negatively affect performance of the patient interface 3000. In further examples, the discontinuous portions of the first connection portion 3900, the second connection portion 3902, or both, as the case may be, may be positioned on the plenum chamber 3200 and the chassis 3500, respectively, to allow multiple, discrete orientations of attachment for the chassis 3500, while preventing unintended orientations. In still further examples, the first connection portion 3900 and the second connection portion 3902 may be circular, and the chassis 3500 and the plenum chamber opening 3202 may also be circular, so that there is no incorrect connection orientation.

In an example of the present technology, the first connection portion 3900 may be joined to the plenum chamber 3200 with adhesive. In a further example, the plenum chamber 3200, e.g., if made from silicone, may be overmolded to the first connection portion 3900. In a still further example, the first connection portion 3900 may be molded in one piece with the plenum chamber 3200.

In another example of the present technology, the second connection portion 3902 may be joined to the chassis 3500 with adhesive. In a further example, the chassis 3500, e.g., if made from silicone, may be overmolded to the second connection portion 3902. In a still further example, the second connection portion 3902 may be molded in one piece with the chassis 3500.

In another example of the present technology, one of the plenum chamber 3200 or the chassis 3500 may include one or more protrusions and the other of the plenum chamber 3200 or the chassis 3500 may include one or more holes corresponding to each protrusion. The protrusions may engage each corresponding hole to releasably secure the chassis 3500 to the plenum chamber 3200. The sealing lip 3800, in this example, would seal around the periphery between the plenum chamber 3200 and the chassis 3500 and the protrusions would fit snugly enough into the holes so as to not allow leakage of air through the holes to atmosphere.

### 4.3.10.3 Sealing lip

Figs. 7A, 10A-10C, 11A-11C, and 13A show examples of the sealing lip 3800. The sealing lip 3800 may be positioned radially around the plenum chamber opening 3202 to seal between the plenum chamber 3200 and the chassis 3500. The sealing lip 3800 may also be radially continuous so to seal around the entire perimeter of the plenum chamber opening 3202. In these examples, the sealing lip 3800 is fixed to the plenum chamber 3200 or the chassis 3500 at a first end, and the sealing lip 3800 is free at a second end. Thus, the sealing lip 3800 may be cantilevered from the chassis 3500 or the plenum chamber 3200. Additionally, the sealing lip 3800 may be curved between the first end and the second end.

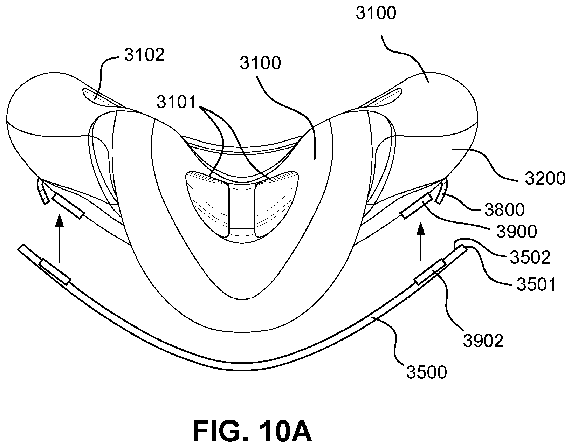

In the example of Figs. 10A-10C, the sealing lip 3800 is fixed to the plenum chamber 3200. Also, the sealing lip 3800 is positioned radially outward of the first connection portion 3900 and the second connection portion 3902. In this arrangement, air pressure 6000 that may leak through the first connection portion 3900 and the second connection portion 3902, when connected, may be prevented by the sealing lip 3800 from reaching ambient so as to maintain the therapeutic pressure, above ambient pressure, within the plenum chamber 3200. In a further example, the sealing lip 3800 may be integrally molded to the plenum chamber 3200. Also in this example, the second connection portion 3902 is shown positioned on the chassis 3500 with a gap G between a peripheral edge 3501 of the chassis 3500 and an outer peripheral edge of the second connection portion 3902. Thus, the peripheral edge 3501 extends farther out radially than the sealing lip 3800 so that the sealing lip 3800 contacts and seals against a lip engagement surface 3502 of the chassis 3500.

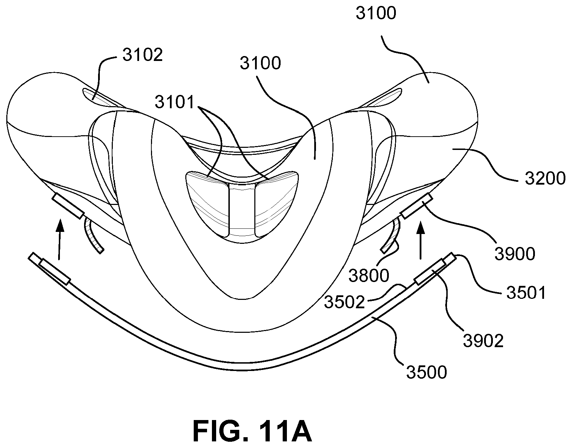

In the example of Figs. 11A-11C, the sealing lip 3800 is fixed to the chassis 3500. Also, the sealing lip 3800 is positioned radially inward of the first connection portion 3900 and the second connection portion 3902. In this arrangement, air pressure 6000 within the plenum chamber 3200 may force the sealing lip 3800 into sealing engagement with chassis 3500 to prevent air leakage to ambient so as to maintain the therapeutic pressure, above ambient pressure, within the plenum chamber 3200. Furthermore, such positioning of the sealing lip 3800 may increase a sealing force of the sealing lip 3800 between the plenum chamber 3200 and the chassis 3500 due to an increase in pressure 6000 within the plenum chamber 3200. In a further example, the sealing lip 3800 may be integrally molded to the chassis 3500. This example may also include the lip engagement surface 3502, but in this case it is positioned radially inward of the first connection portion 3900 and second connection portion 3902, as is the sealing lip 3800.

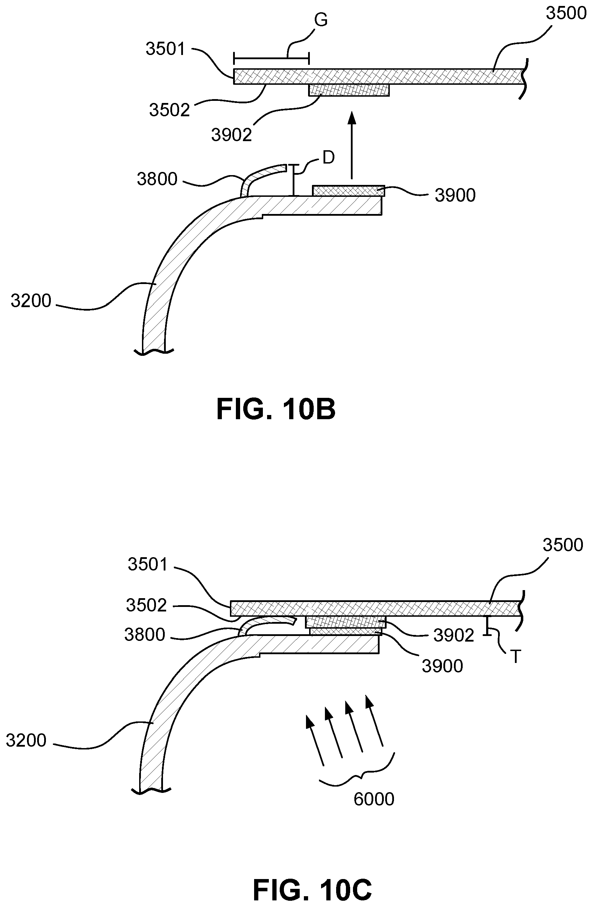

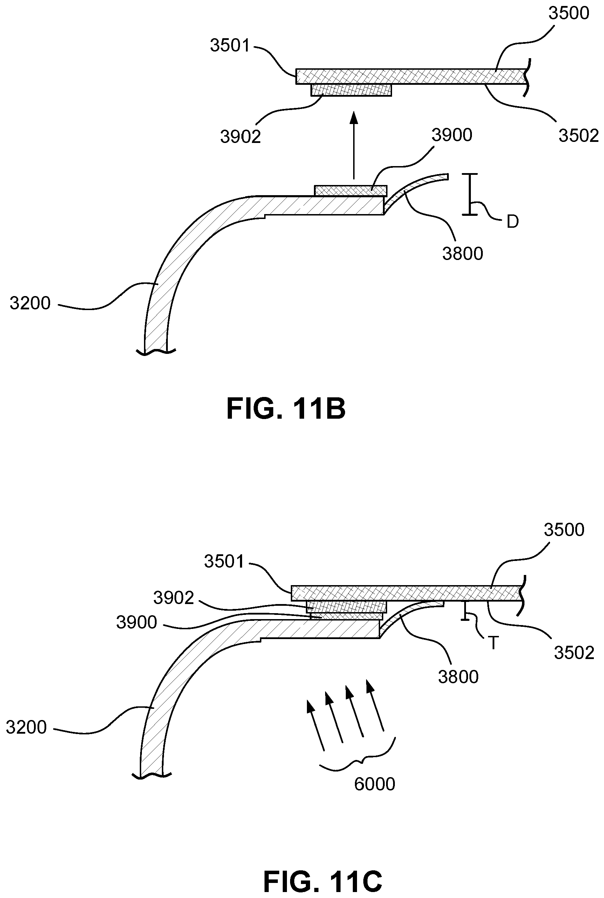

Figs. 10B, 10C, 11B, and 11C show examples of the sealing lip 3800 having, in an undeformed state, a dimension D extending from the plenum chamber 3200 or the chassis 3500, respectively, in a direction that is orthogonal to a surface of the plenum chamber 3200 or the chassis 3500, respectively, to which the sealing lip is attached. The dimension D may be greater than a combined thickness T of the first connection portion 3900 and the second connection portion 3902 so that when the plenum chamber 3200 and the chassis 3500 are connected, the sealing lip 3800 may be deformed so as to seal off the gap between the plenum chamber 3200 and the chassis 3500. Additionally, because the sealing lip 3800 extends farther from the plenum chamber 3200 or the chassis 3500, as the case may be, the sealing lip 3800 will contact and seal against the other of the plenum chamber 3200 or the chassis 3500 before the first connection portion 3900 and the second connection portion 3902 are connected.

In examples, the sealing lip 3800 may be constructed from an elastomeric material. In further examples, the sealing lip 3800 may be constructed from silicone.

In the examples depicted in Figs. 10B, 10C, 11B, and 11C, it can also be seen that the sealing lip 3800 is positioned relative to the first connection portion 3900 and the second connection portion 3902 so that these components do not interfere with one another's respective functions when the chassis 3500 is connected to the plenum chamber 3200.

### 4.4 RPT DEVICE

An RPT device 4000 in accordance with one aspect of the present technology comprises mechanical, pneumatic, and/or electrical components and is configured to execute one or more algorithms, such as any of the methods, in whole or in part, described herein. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to +150 L/min while maintaining a positive pressure of at least 6 cmH2O, or at least 10cmH2O, or at least 20 cmH2O.

The RPT device may have an external housing 4010, formed in two parts, an upper portion 4012 and a lower portion 4014. Furthermore, the external housing 4010 may include one or more panel(s) 4015. The RPT device 4000 comprises a chassis 4016 that supports one or more internal components of the RPT device 4000. The RPT device 4000 may include a handle 4018.

The pneumatic path of the RPT device 4000 may comprise one or more air path items, e.g., an inlet air filter 4112, an inlet muffler 4122, a pressure generator 4140 capable of supplying air at positive pressure (e.g., a blower 4142), an outlet muffler 4124 and one or more transducers 4270, such as pressure sensors and flow rate sensors.

One or more of the air path items may be located within a removable unitary structure which will be referred to as a pneumatic block 4020. The pneumatic block 4020 may be located within the external housing 4010. In one form a pneumatic block 4020 is supported by, or formed as part of the chassis 4016.

The RPT device 4000 may have an electrical power supply 4210, one or more input devices 4220, a central controller, a therapy device controller, a pressure generator 4140, one or more protection circuits, memory, transducers 4270, data communication interface and one or more output devices. Electrical components 4200 may be mounted on a single Printed Circuit Board Assembly (PCBA) 4202. In an alternative form, the RPT device 4000 may include more than one PCBA 4202.

### 4.4.1 RPT device mechanical & pneumatic components

An RPT device may comprise one or more of the following components in an integral unit. In an alternative form, one or more of the following components may be located as respective separate units.

### 4.4.1.1 Air filter(s)

An RPT device in accordance with one form of the present technology may include an air filter 4110, or a plurality of air filters 4110.

In one form, an inlet air filter 4112 is located at the beginning of the pneumatic path upstream of a pressure generator 4140.

In one form, an outlet air filter 4114, for example an antibacterial filter, is located between an outlet of the pneumatic block 4020 and a patient interface 3000.

### 4.4.1.2 Muffler(s)

An RPT device in accordance with one form of the present technology may include a muffler 4120, or a plurality of mufflers 4120.

In one form of the present technology, an inlet muffler 4122 is located in the pneumatic path upstream of a pressure generator 4140.

In one form of the present technology, an outlet muffler 4124 is located in the pneumatic path between the pressure generator 4140 and a patient interface 3000.

### 4.4.1.3 Pressure generator

In one form of the present technology, a pressure generator 4140 for producing a flow, or a supply, of air at positive pressure is a controllable blower 4142. For example, the blower 4142 may include a brushless DC motor 4144 with one or more impellers. The impellers may be located in a volute. The blower may be capable of delivering a supply of air, for example at a rate of up to about 120 litres/minute, at a positive pressure in a range from about 4 cmH2O to about 20 cmH2O, or in other forms up to about 30 cmH2O when delivering respiratory pressure therapy. The blower may be as described in any one of the following patents or patent applications: U.S. Patent No. 7,866,944; U.S. Patent No. 8,638,014; U.S. Patent No. 8,636,479; and PCT Patent Application Publication No. WO 2013/020167.

### 4.4.1.4 Anti-spill back valve

In one form of the present technology, an anti-spill back valve 4160 is located between the humidifier 5000 and the pneumatic block 4020. The anti-spill back valve is constructed and arranged to reduce the risk that water will flow upstream from the humidifier 5000, for example to the motor 4144.

### 4.5 AIR CIRCUIT

An air circuit 4170 in accordance with an aspect of the present technology is a conduit or a tube constructed and arranged to allow, in use, a flow of air to travel between two components such as RPT device 4000 and the patient interface 3000.

In particular, the air circuit 4170 may be in fluid connection with the outlet of the pneumatic block 4020 and the patient interface. The air circuit may be referred to as an air delivery tube. In some cases there may be separate limbs of the circuit for inhalation and exhalation. In other cases a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. One example of an air circuit 4170 comprising a heated wire circuit is described in United States Patent 8,733,349.

### 4.5.1 Supplementary gas delivery

In one form of the present technology, supplementary gas, e.g. oxygen, 4180 is delivered to one or more points in the pneumatic path, such as upstream of the pneumatic block 4020, to the air circuit 4170, and/or to the patient interface 3000.

### 4.6 HUMIDIFIER

### 4.6.1 Humidifier overview

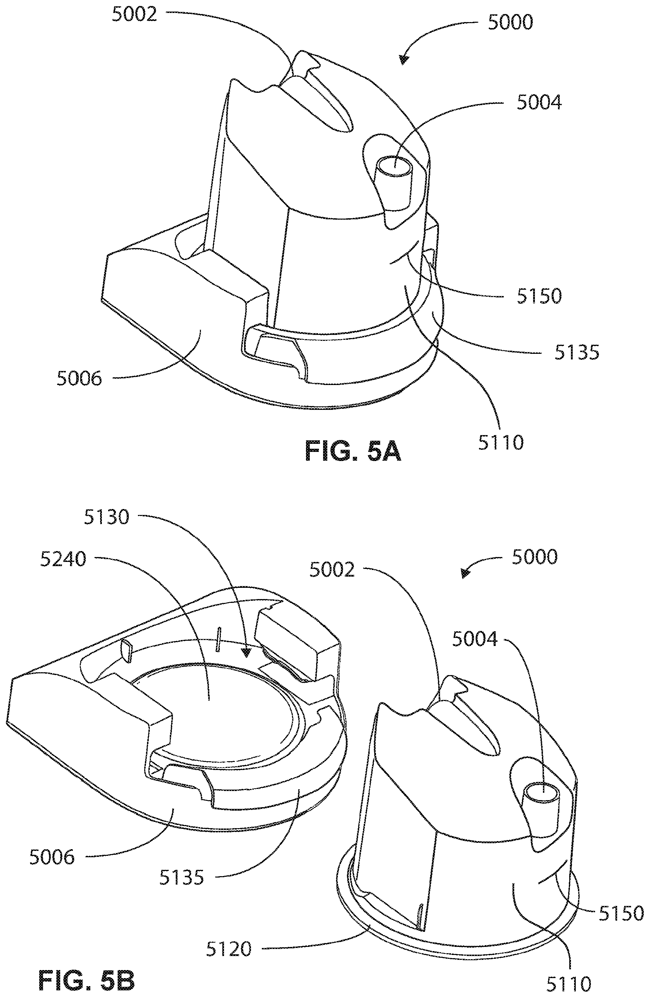

In one form of the present technology there is provided a humidifier 5000 (e.g. as shown in Fig. 5A) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

The humidifier 5000 may comprise a humidifier reservoir 5110, a humidifier inlet 5002 to receive a flow of air, and a humidifier outlet 5004 to deliver a humidified flow of *air*. In some forms, as shown in Fig. 5A and Fig. 5B, an inlet and an outlet of the humidifier reservoir 5110 may be the humidifier inlet 5002 and the humidifier outlet 5004 respectively. The humidifier 5000 may further comprise a humidifier base 5006, which may be adapted to receive the humidifier reservoir 5110 and comprise a heating element 5240.

### 4.6.2 Humidifier components

### 4.6.2.1 Water reservoir

According to one arrangement, the humidifier 5000 may comprise a water reservoir 5110 configured to hold, or retain, a volume of liquid (e.g. water) to be evaporated for humidification of the flow of air. The water reservoir 5110 may be configured to hold a predetermined maximum volume of water in order to provide adequate humidification for at least the duration of a respiratory therapy session, such as one evening of sleep. Typically, the reservoir 5110 is configured to hold several hundred millilitres of water, e.g. 300 millilitres (ml), 325 ml, 350 ml or 400 ml. In other forms, the humidifier 5000 may be configured to receive a supply of water from an external water source such as a building's water supply system.

According to one aspect, the water reservoir 5110 is configured to add humidity to a flow of air from the RPT device 4000 as the flow of air travels therethrough. In one form, the water reservoir 5110 may be configured to encourage the flow of air to travel in a tortuous path through the reservoir 5110 while in contact with the volume of water therein.

According to one form, the reservoir 5110 may be removable from the humidifier 5000, for example in a lateral direction as shown in Fig. 5A and Fig. 5B.

The reservoir 5110 may also be configured to discourage egress of liquid therefrom, such as when the reservoir 5110 is displaced and/or rotated from its normal, working orientation, such as through any apertures and/or in between its subcomponents. As the flow of air to be humidified by the humidifier 5000 is typically pressurised, the reservoir 5110 may also be configured to prevent losses in pneumatic pressure through leak and/or flow impedance.

### 4.6.2.2 Conductive portion

According to one arrangement, the reservoir 5110 comprises a conductive portion 5120 configured to allow efficient transfer of heat from the heating element 5240 to the volume of liquid in the reservoir 5110. In one form, the conductive portion 5120 may be arranged as a plate, although other shapes may also be suitable. All or a part of the conductive portion 5120 may be made of a thermally conductive material such as aluminium (e.g. approximately 2 mm thick, such as 1 mm, 1.5 mm, 2.5 mm or 3 mm), another heat conducting metal or some plastics. In some cases, suitable heat conductivity may be achieved with less conductive materials of suitable geometry.

### 4.6.2.3 Humidifier reservoir dock

In one form, the humidifier 5000 may comprise a humidifier reservoir dock 5130 (as shown in Fig. 5B) configured to receive the humidifier reservoir 5110. In some arrangements, the humidifier reservoir dock 5130 may comprise a locking feature such as a locking lever 5135 configured to retain the reservoir 5110 in the humidifier reservoir dock 5130.

### 4.6.2.4 Water level indicator

The humidifier reservoir 5110 may comprise a water level indicator 5150 as shown in Fig. 5A-5B. In some forms, the water level indicator 5150 may provide one or more indications to a user such as the patient 1000 or a care giver regarding a quantity of the volume of water in the humidifier reservoir 5110. The one or more indications provided by the water level indicator 5150 may include an indication of a maximum, predetermined volume of water, any portions thereof, such as 25%, 50% or 75% or volumes such as 200 ml, 300 ml or 400ml.

### 4.7 BREATHING WAVEFORMS

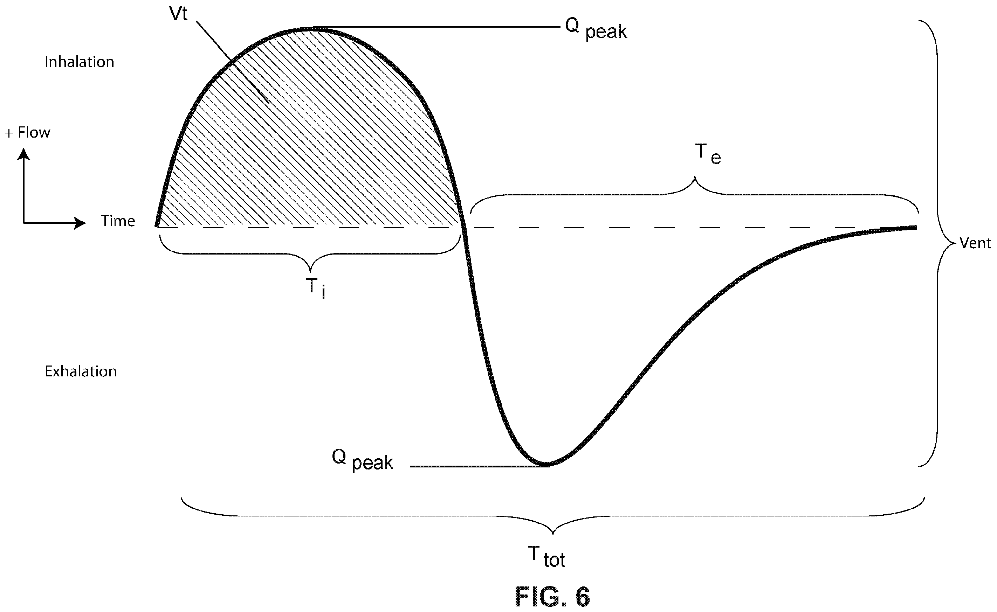

Fig. 6 shows a model typical breath waveform of a person while sleeping. The horizontal axis is time, and the vertical axis is respiratory flow rate. While the parameter values may vary, a typical breath may have the following approximate values: tidal volume *Vt* 0.5L, inhalation time *Ti* 1.6s, peak inspiratory flow rate *Qpeak* 0.4 L/s, exhalation time *Te* 2.4s, peak expiratory flow rate *Qpeak* -0.5 L/s. The total duration of the breath, *Ttot*, is about 4s. The person typically breathes at a rate of about 15 breaths per minute (BPM), with Ventilation *Vent* about 7.5 L/min. A typical duty cycle, the ratio of *Ti* to *Ttot*, is about 40%.

### 4.8 GLOSSARY

For the purposes of the present technology disclosure, in certain forms of the present technology, one or more of the following definitions may apply. In other forms of the present technology, alternative definitions may apply.

### 4.8.1 General

*Air*: In certain forms of the present technology, air may be taken to mean atmospheric air, and in other forms of the present technology air may be taken to mean some other combination of breathable gases, e.g. oxygen enriched air.

*Ambient*: In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy:* CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy:* Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate*: The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol Q. 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Device flow rate, *Qd,* is the flow rate of air leaving the RPT device. Total flow rate, *Qt,* is the flow rate of air and any supplementary gas reaching the patient interface via the air circuit. Vent flow rate, *Qv,* is the flow rate of air leaving a vent to allow washout of exhaled gases. Leak flow rate, *Ql,* is the flow rate of leak from a patient interface system or elsewhere. Respiratory flow rate, *Qr,* is the flow rate of air that is received into the patient's respiratory system.

*Flow therapy*: Respiratory therapy comprising the delivery of a flow of air to an entrance to the airways at a controlled flow rate referred to as the treatment flow rate that is typically positive throughout the patient's breathing cycle.

*Humidifier*: The word humidifier will be taken to mean a humidifying apparatus constructed and arranged, or configured with a physical structure to be capable of providing a therapeutically beneficial amount of water (H₂O) vapour to a flow of air to ameliorate a medical respiratory condition of a patient.

*Leak*: The word leak will be taken to be an unintended flow of air. In one example, leak may occur as the result of an incomplete seal between a mask and a patient's face. In another example leak may occur in a swivel elbow to the ambient.

*Noise, conducted (acoustic)*: Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic)*: Radiated noise in the present document refers to noise which is carried to the patient by the ambient air. In one form, radiated noise may be quantified by measuring sound power/pressure levels of the object in question according to ISO 3744.

*Noise, vent (acoustic)*: Vent noise in the present document refers to noise which is generated by the flow of air through any vents such as vent holes of the patient interface.

*Oxygen enriched air*: Air with a concentration of oxygen greater than that of atmospheric air (21%), for example at least about 50% oxygen, at least about 60% oxygen, at least about 70% oxygen, at least about 80% oxygen, at least about 90% oxygen, at least about 95% oxygen, at least about 98% oxygen, or at least about 99% oxygen. "Oxygen enriched air" is sometimes shortened to "oxygen".

*Medical Oxygen*: Medical oxygen is defined as oxygen enriched air with an oxygen concentration of 80% or greater.

*Patient*: A person, whether or not they are suffering from a respiratory condition.

*Pressure*: Force per unit area. Pressure may be expressed in a range of units, including cmH₂O, g-f/cm² and hectopascal. 1 cmH₂O is equal to 1 g-f/cm² and is approximately 0.98 hectopascal (1 hectopascal = 100 Pa = 100 N/m² = 1 millibar ~ 0.001 atm). In this specification, unless otherwise stated, pressure is given in units of cmH₂O.

The pressure in the patient interface is given the symbol *Pm,* while the treatment pressure, which represents a target value to be achieved by the interface pressure *Pm* at the current instant of time, is given the symbol *Pt.*

*Respiratory Pressure Therapy*: The application of a supply of air to an entrance to the airways at a treatment pressure that is typically positive with respect to atmosphere.

*Ventilator*: A mechanical device that provides pressure support to a patient to perform some or all of the work of breathing.

### 4.8.1.1 Materials

*Silicone or Silicone Elastomer*: A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker.

*Polycarbonate*: a thermoplastic polymer of Bisphenol-A Carbonate.

### 4.8.1.2 Mechanical properties

*Resilience*: Ability of a material to absorb energy when deformed elastically and to release the energy upon unloading.

*Resilient*: Will release substantially all of the energy when unloaded. Includes e.g. certain silicones, and thermoplastic elastomers.

*Hardness*: The ability of a material per se to resist deformation (e.g. described by a Young's Modulus, or an indentation hardness scale measured on a standardised sample size).
- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness* (or *rigidity*) of a structure or component: The ability of the structure or component to resist deformation in response to an applied load. The load may be a force or a moment, e.g. compression, tension, bending or torsion. The structure or component may offer different resistances in different directions. The inverse of stiffness is flexibility.

*Floppy* structure or component: A structure or component that will change shape, e.g. bend, when caused to support its own weight, within a relatively short period of time such as 1 second.

*Rigid* structure or component: A structure or component that will not substantially change shape when subject to the loads typically encountered in use. An example of such a use may be setting up and maintaining a patient interface in sealing relationship with an entrance to a patient's airways, e.g. at a load of approximately 20 to 30 cmH₂O pressure.

As an example, an I-beam may comprise a different bending stiffness (resistance to a bending load) in a first direction in comparison to a second, orthogonal direction. In another example, a structure or component may be floppy in a first direction and rigid in a second direction.

### 4.8.2 Respiratory cycle

*Apnea*: According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

*Breathing rate*: The rate of spontaneous respiration of a patient, usually measured in breaths per minute.

*Duty cycle*: The ratio of inhalation time, Ti to total breath time, Ttot.

*Effort* (breathing): The work done by a spontaneously breathing person attempting to breathe.

*Expiratory portion* of a breathing cycle: The period from the start of expiratory flow to the start of inspiratory flow.

*Flow limitation*: Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

Types of flow limited inspiratory waveforms:
(i) Flattened: Having a rise followed by a relatively flat portion, followed by a fall.
(ii) M-shaped: Having two local peaks, one at the leading edge, and one at the trailing edge, and a relatively flat portion between the two peaks.
(iii) Chair-shaped: Having a single local peak, the peak being at the leading edge, followed by a relatively flat portion.
(iv) Reverse-chair shaped: Having a relatively flat portion followed by single local peak, the peak being at the trailing edge.

*Hypopnea*: According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:
(i) a 30% reduction in patient breathing for at least 10 seconds plus an associated 4% desaturation; or
(ii) a reduction in patient breathing (but less than 50%) for at least 10 seconds, with an associated desaturation of at least 3% or an arousal.

*Hyperpnea*: An increase in flow to a level higher than normal.

*Inspiratory portion* of a breathing cycle: The period from the start of inspiratory flow to the start of expiratory flow will be taken to be the inspiratory portion of a breathing cycle.

*Patency* (airway): The degree of the airway being open, or the extent to which the airway is open. A patent airway is open. Airway patency may be quantified, for example with a value of one (1) being patent, and a value of zero (0), being closed (obstructed).

*Positive End-Expiratory Pressure (PEEP)*: The pressure above atmosphere in the lungs that exists at the end of expiration.

*Peak flow rate (Qpeak)*: The maximum value of flow rate during the inspiratory portion of the respiratory flow waveform.

*Respiratory flow rate, patient airflow rate, respiratory airflow rate (Qr)*: These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

*Tidal volume (Vt)*: The volume of air inhaled or exhaled during normal breathing, when extra effort is not applied. In principle the inspiratory volume *Vi* (the volume of air inhaled) is equal to the expiratory volume Ve (the volume of air exhaled), and therefore a single tidal volume *Vt* may be defined as equal to either quantity. In practice the tidal volume *Vt* is estimated as some combination, e.g. the mean, of the inspiratory volume Vi and the expiratory volume *Ve*.

*Inhalation Time* (*Ti*): The duration of the inspiratory portion of the respiratory flow rate waveform.

*Exhalation Time* (*Te*): The duration of the expiratory portion of the respiratory flow rate waveform.

*Total Time* (*Ttot*): The total duration between the start of one inspiratory portion of a respiratory flow rate waveform and the start of the following inspiratory portion of the respiratory flow rate waveform.

*Typical recent ventilation*: The value of ventilation around which recent values of ventilation Vent over some predetermined timescale tend to cluster, that is, a measure of the central tendency of the recent values of ventilation.

*Upper airway obstruction (UAO)*: includes both partial and total upper airway obstruction. This may be associated with a state of flow limitation, in which the flow rate increases only slightly or may even decrease as the pressure difference across the upper airway increases (Starling resistor behaviour).

*Ventilation (Vent)*: A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 4.8.3 Anatomy

### 4.8.3.1 Anatomy of the face

Ala: the external outer wall or "wing" of each nostril (plural: alar)

Alare: The most lateral point on the nasal ala.

Alar curvature (or alar crest) point: The most posterior point in the curved base line of each ala, found in the crease formed by the union of the ala with the cheek.

Auricle: The whole external visible part of the ear.

(nose) Bony framework: The bony framework of the nose comprises the nasal bones, the frontal process of the maxillae and the nasal part of the frontal bone.

(nose) Cartilaginous framework: The cartilaginous framework of the nose comprises the septal, lateral, major and minor cartilages.

Columella: the strip of skin that separates the nares and which runs from the pronasale to the upper lip.

Columella angle: The angle between the line drawn through the midpoint of the nostril aperture and a line drawn perpendicular to the Frankfort horizontal while intersecting subnasale.

Frankfort horizontal plane: A line extending from the most inferior point of the orbital margin to the left tragion. The tragion is the deepest point in the notch superior to the tragus of the auricle.

Glabella: Located on the soft tissue, the most prominent point in the midsagittal plane of the forehead.

Lateral nasal cartilage: A generally triangular plate of cartilage. Its superior margin is attached to the nasal bone and frontal process of the maxilla, and its inferior margin is connected to the greater alar cartilage.

Greater alar cartilage: A plate of cartilage lying below the lateral nasal cartilage. It is curved around the anterior part of the naris. Its posterior end is connected to the frontal process of the maxilla by a tough fibrous membrane containing three or four minor cartilages of the ala.

Nares (Nostrils): Approximately ellipsoidal apertures forming the entrance to the nasal cavity. The singular form of nares is naris (nostril). The nares are separated by the nasal septum.

Naso-labial sulcus or Naso-labial fold: The skin fold or groove that runs from each side of the nose to the corners of the mouth, separating the cheeks from the upper lip.

Naso-labial angle: The angle between the columella and the upper lip, while intersecting subnasale.

Otobasion inferior: The lowest point of attachment of the auricle to the skin of the face.

Otobasion superior: The highest point of attachment of the auricle to the skin of the face.

Pronasale: the most protruded point or tip of the nose, which can be identified in lateral view of the rest of the portion of the head.

Philtrum: the midline groove that runs from lower border of the nasal septum to the top of the lip in the upper lip region.

Pogonion: Located on the soft tissue, the most anterior midpoint of the chin.

Ridge (nasal): The nasal ridge is the midline prominence of the nose, extending from the Sellion to the Pronasale.

Sagittal plane: A vertical plane that passes from anterior (front) to posterior (rear). The midsagittal plane is a sagittal plane that divides the body into right and left halves.

Sellion: Located on the soft tissue, the most concave point overlying the area of the frontonasal suture.

Septal cartilage (nasal): The nasal septal cartilage forms part of the septum and divides the front part of the nasal cavity.

Subalare: The point at the lower margin of the alar base, where the alar base joins with the skin of the superior (upper) lip.

Subnasal point: Located on the soft tissue, the point at which the columella merges with the upper lip in the midsagittal plane.

Supramenton: The point of greatest concavity in the midline of the lower lip between labrale inferius and soft tissue pogonion

### 4.8.3.2 Anatomy of the skull

Frontal bone: The frontal bone includes a large vertical portion, the squama frontalis, corresponding to the region known as the forehead.

Mandible: The mandible forms the lower jaw. The mental protuberance is the bony protuberance of the jaw that forms the chin.

Maxilla: The maxilla forms the upper jaw and is located above the mandible and below the orbits. The frontal process of the maxilla projects upwards by the side of the nose, and forms part of its lateral boundary.

Nasal bones: The nasal bones are two small oblong bones, varying in size and form in different individuals; they are placed side by side at the middle and upper part of the face, and form, by their junction, the "bridge" of the nose.

Nasion: The intersection of the frontal bone and the two nasal bones, a depressed area directly between the eyes and superior to the bridge of the nose.

Occipital bone: The occipital bone is situated at the back and lower part of the cranium. It includes an oval aperture, the foramen magnum, through which the cranial cavity communicates with the vertebral canal. The curved plate behind the foramen magnum is the squama occipitalis.

Orbit: The bony cavity in the skull to contain the eyeball.

Parietal bones: The parietal bones are the bones that, when joined together, form the roof and sides of the cranium.

Temporal bones: The temporal bones are situated on the bases and sides of the skull, and support that part of the face known as the temple.

Zygomatic bones: The face includes two zygomatic bones, located in the upper and lateral parts of the face and forming the prominence of the cheek.

### 4.8.3.3 Anatomy of the respiratory system

Diaphragm: A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

Larynx: The larynx, or voice box houses the vocal folds and connects the inferior part of the pharynx (hypopharynx) with the trachea.

Lungs: The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

Nasal cavity: The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

Pharynx: The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 4.8.4 Patient interface

Anti-asphyxia valve (AAV): The component or sub-assembly of a mask system that, by opening to atmosphere in a failsafe manner, reduces the risk of excessive CO2 rebreathing by a patient.

Elbow: An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

Frame: Frame will be taken to mean a mask structure that bears the load of tension between two or more points of connection with a headgear. A mask frame may be a non-airtight load bearing structure in the mask. However, some forms of mask frame may also be air-tight.

Functional dead space: (description to be inserted here)

Headgear: Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

Membrane: Membrane will be taken to mean a typically thin element that has, preferably, substantially no resistance to bending, but has resistance to being stretched.

Plenum chamber: a mask plenum chamber will be taken to mean a portion of a patient interface having walls at least partially enclosing a volume of space, the volume having air therein pressurised above atmospheric pressure in use. A shell may form part of the walls of a mask plenum chamber.

Seal: May be a noun form ("a seal") which refers to a structure, or a verb form ("to seal") which refers to the effect. Two elements may be constructed and/or arranged to 'seal' or to effect 'sealing' therebetween without requiring a separate 'seal' element per se.

Shell: A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

Stiffener: A stiffener will be taken to mean a structural component designed to increase the bending resistance of another component in at least one direction.

Strut: A strut will be taken to be a structural component designed to increase the compression resistance of another component in at least one direction.

Swivel (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

Tie (noun): A structure designed to resist tension.

Vent: (noun): A structure that allows a flow of air from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

### 4.8.5 Shape of structures

Products in accordance with the present technology may comprise one or more three-dimensional mechanical structures, for example a mask cushion or an impeller. The three-dimensional structures may be bounded by two-dimensional surfaces. These surfaces may be distinguished using a label to describe an associated surface orientation, location, function, or some other characteristic. For example a structure may comprise one or more of an anterior surface, a posterior surface, an interior surface and an exterior surface. In another example, a seal-forming structure may comprise a face-contacting (e.g. outer) surface, and a separate non-face-contacting (e.g. underside or inner) surface. In another example, a structure may comprise a first surface and a second surface.

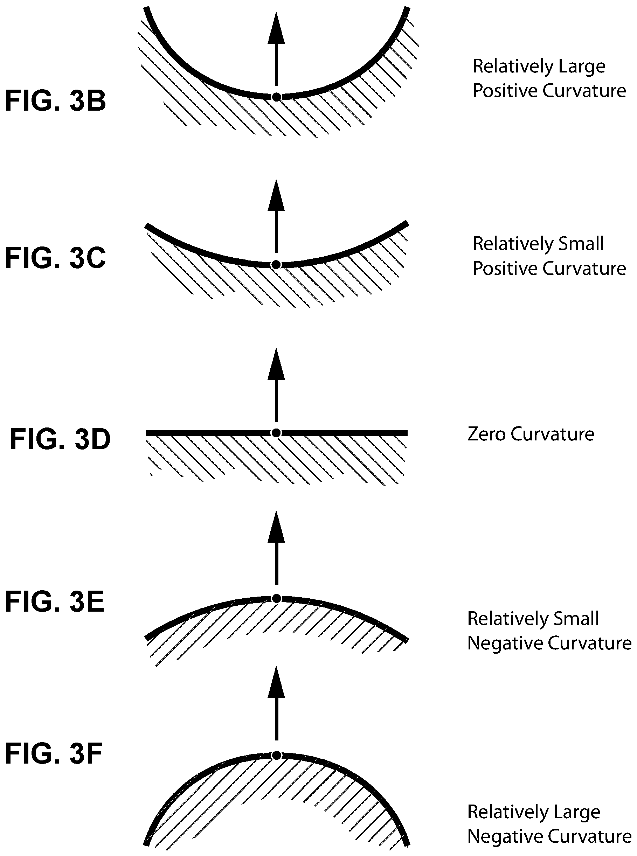

To facilitate describing the shape of the three-dimensional structures and the surfaces, we first consider a cross-section through a surface of the structure at a point, *p.* See Fig. 3B to Fig. 3F, which illustrate examples of cross-sections at point *p* on a surface, and the resulting plane curves. Figs. 3B to 3F also illustrate an outward normal vector at *p.* The outward normal vector at *p* points away from the surface. In some examples we describe the surface from the point of view of an imaginary small person standing upright on the surface.

### 4.8.5.1 Curvature in one dimension

The curvature of a plane curve at p may be described as having a sign (e.g. positive, negative) and a magnitude (e.g. 1/radius of a circle that just touches the curve at p).

Positive curvature: If the curve at p turns towards the outward normal, the curvature at that point will be taken to be positive (if the imaginary small person leaves the point p they must walk uphill). See Fig. 3B (relatively large positive curvature compared to Fig. 3C) and Fig. 3C (relatively small positive curvature compared to Fig. 3B). Such curves are often referred to as concave.

Zero curvature: If the curve at p is a straight line, the curvature will be taken to be zero (if the imaginary small person leaves the point p, they can walk on a level, neither up nor down). See Fig. 3D.

Negative curvature: If the curve at p turns away from the outward normal, the curvature in that direction at that point will be taken to be negative (if the imaginary small person leaves the point p they must walk downhill). See Fig. 3E (relatively small negative curvature compared to Fig. 3F) and Fig. 3F (relatively large negative curvature compared to Fig. 3E). Such curves are often referred to as convex.

### 4.8.5.2 Curvature of two dimensional surfaces

A description of the shape at a given point on a two-dimensional surface in accordance with the present technology may include multiple normal cross-sections. The multiple cross-sections may cut the surface in a plane that includes the outward normal (a "normal plane"), and each cross-section may be taken in a different direction. Each cross-section results in a plane curve with a corresponding curvature. The different curvatures at that point may have the same sign, or a different sign. Each of the curvatures at that point has a magnitude, e.g. relatively small. The plane curves in Figs. 3B to 3F could be examples of such multiple cross-sections at a particular point.

Principal curvatures and directions: The directions of the normal planes where the curvature of the curve takes its maximum and minimum values are called the principal directions. In the examples of Fig. 3B to Fig. 3F, the maximum curvature occurs in Fig. 3B, and the minimum occurs in Fig. 3F, hence Fig. 3B and Fig. 3F are cross sections in the principal directions. The principal curvatures at p are the curvatures in the principal directions.

Region of a surface: A connected set of points on a surface. The set of points in a region may have similar characteristics, e.g. curvatures or signs.

Saddle region: A region where at each point, the principal curvatures have opposite signs, that is, one is positive, and the other is negative (depending on the direction to which the imaginary person turns, they may walk uphill or downhill).

Dome region: A region where at each point the principal curvatures have the same sign, e.g. both positive (a "concave dome") or both negative (a "convex dome").

Cylindrical region: A region where one principal curvature is zero (or, for example, zero within manufacturing tolerances) and the other principal curvature is non-zero.

Planar region: A region of a surface where both of the principal curvatures are zero (or, for example, zero within manufacturing tolerances).

Edge of a surface: A boundary or limit of a surface or region.

Path: In certain forms of the present technology, 'path' will be taken to mean a path in the mathematical - topological sense, e.g. a continuous space curve from f(0) to f(1) on a surface. In certain forms of the present technology, a 'path' may be described as a route or course, including e.g. a set of points on a surface. (The path for the imaginary person is where they walk on the surface, and is analogous to a garden path).

Path length: In certain forms of the present technology, 'path length' will be taken to mean the distance along the surface from f(0) to f(1), that is, the distance along the path on the surface. There may be more than one path between two points on a surface and such paths may have different path lengths. (The path length for the imaginary person would be the distance they have to walk on the surface along the path).

Straight-line distance: The straight-line distance is the distance between two points on a surface, but without regard to the surface. On planar regions, there would be a path on the surface having the same path length as the straight-line distance between two points on the surface. On non-planar surfaces, there may be no paths having the same path length as the straight-line distance between two points. (For the imaginary person, the straight-line distance would correspond to the distance 'as the crow flies'.)

### 4.8.5.3 Space curves

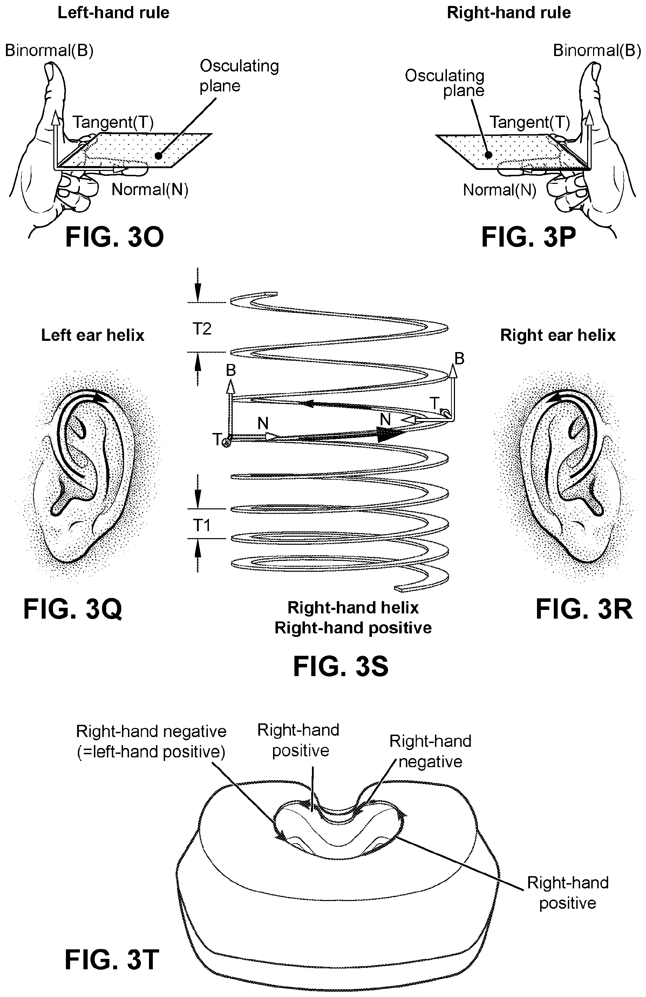

Space curves: Unlike a plane curve, a space curve does not necessarily lie in any particular plane. A space curve may be closed, that is, having no endpoints. A space curve may be considered to be a one-dimensional piece of three-dimensional space. An imaginary person walking on a strand of the DNA helix walks along a space curve. A typical human left ear comprises a helix, which is a left-hand helix, see Fig. 3Q. A typical human right ear comprises a helix, which is a right-hand helix, see Fig. 3R. Fig. 3S shows a right-hand helix. The edge of a structure, e.g. the edge of a membrane or impeller, may follow a space curve. In general, a space curve may be described by a curvature and a torsion at each point on the space curve. Torsion is a measure of how the curve turns out of a plane. Torsion has a sign and a magnitude. The torsion at a point on a space curve may be characterised with reference to the tangent, normal and binormal vectors at that point.

Tangent unit vector (or unit tangent vector): For each point on a curve, a vector at the point specifies a direction from that point, as well as a magnitude. A tangent unit vector is a unit vector pointing in the same direction as the curve at that point. If an imaginary person were flying along the curve and fell off her vehicle at a particular point, the direction of the tangent vector is the direction she would be travelling.

Unit normal vector: As the imaginary person moves along the curve, this tangent vector itself changes. The unit vector pointing in the same direction that the tangent vector is changing is called the unit principal normal vector. It is perpendicular to the tangent vector.

Binormal unit vector: The binormal unit vector is perpendicular to both the tangent vector and the principal normal vector. Its direction may be determined by a right-hand rule (see e.g. Fig. 3P), or alternatively by a left-hand rule (Fig. 3O).

Osculating plane: The plane containing the unit tangent vector and the unit principal normal vector. See Figures 3O and 3P.

Torsion of a space curve: The torsion at a point of a space curve is the magnitude of the rate of change of the binormal unit vector at that point. It measures how much the curve deviates from the osculating plane. A space curve which lies in a plane has zero torsion. A space curve which deviates a relatively small amount from the osculating plane will have a relatively small magnitude of torsion (e.g. a gently sloping helical path). A space curve which deviates a relatively large amount from the osculating plane will have a relatively large magnitude of torsion (e.g. a steeply sloping helical path). With reference to Fig. 3S, since T2>T1, the magnitude of the torsion near the top coils of the helix of Fig. 3S is greater than the magnitude of the torsion of the bottom coils of the helix of Fig. 3S

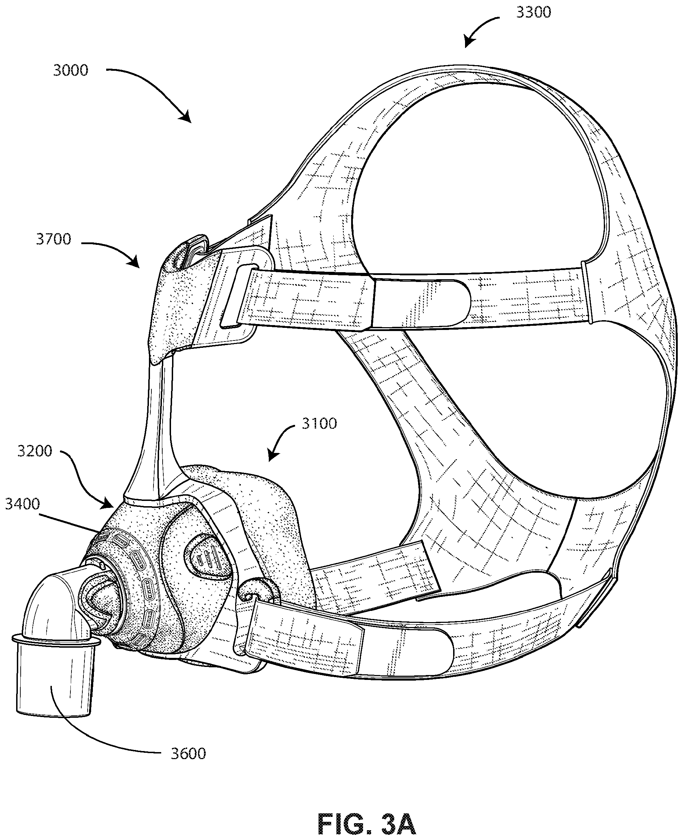

With reference to the right-hand rule of Fig. 3P, a space curve turning towards the direction of the right-hand binormal may be considered as having a right-hand positive torsion (e.g. a right-hand helix as shown in Fig. 3S). A space curve turning away from the direction of the right-hand binormal may be considered as having a right-hand negative torsion (e.g. a left-hand helix).

Equivalently, and with reference to a left-hand rule (see Fig. 3O), a space curve turning towards the direction of the left-hand binormal may be considered as having a left-hand positive torsion (e.g. a left-hand helix). Hence left-hand positive is equivalent to right-hand negative. See Fig. 3T.

### 4.8.5.4 Holes

A surface may have a one-dimensional hole, e.g. a hole bounded by a plane curve or by a space curve. Thin structures (e.g. a membrane) with a hole, may be described as having a one-dimensional hole. See for example the one dimensional hole in the surface of structure shown in Fig. 3I, bounded by a plane curve.

A structure may have a two-dimensional hole, e.g. a hole bounded by a surface. For example, an inflatable tyre has a two dimensional hole bounded by the interior surface of the tyre. In another example, a bladder with a cavity for air or gel could have a two-dimensional hole. See for example the cushion of Fig. 3L and the example cross-sections therethrough in Fig. 3M and Fig. 3N, with the interior surface bounding a two dimensional hole indicated. In a yet another example, a conduit may comprise a one-dimension hole (e.g. at its entrance or at its exit), and a two-dimension hole bounded by the inside surface of the conduit. See also the two dimensional hole through the structure shown in Fig. 3K, bounded by a surface as shown.

### 4.9 OTHER REMARKS

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the scope of the technology The invention is defined by the claims which follow.

### 4.10 REFERENCE SIGNS LIST

| | |
|---|---|
| patient | 1000 |
| bed partner | 1100 |
| patient interface | 3000 |
| seal - forming structure | 3100 |
| nasal hole | 3101 |
| oral hole | 3102 |
| plenum chamber | 3200 |
| plenum chamber opening | 3202 |
| chord | 3210 |
| superior point | 3220 |
| inferior point | 3230 |
| positioning and stabilising structure | 3300 |
| vent | 3400 |
| chassis | 3500 |
| peripheral edge | 3501 |
| lip engagement surface | 3502 |
| tie attachment structure | 3510 |
| connection port | 3600 |
| forehead support | 3700 |
| sealing lip | 3800 |
| first connection portion | 3900 |
| second connection portion | 3902 |
| RPT device | 4000 |
| external housing | 4010 |
| upper portion | 4012 |
| lower portion | 4014 |
| panel | 4015 |
| chassis | 4016 |
| handle | 4018 |
| pneumatic block | 4020 |
| air filter | 4110 |
| inlet air filter | 4112 |
| outlet air filter | 4114 |
| muffler | 4120 |
| inlet muffler | 4122 |
| outlet muffler | 4124 |
| pressure generator | 4140 |
| blower | 4142 |
| motor | 4144 |
| anti - spill back valve | 4160 |
| air circuit | 4170 |
| electrical components | 4200 |
| PCBA | 4202 |
| electrical power supply | 4210 |
| input devices | 4220 |
| transducers | 4270 |
| humidifier | 5000 |
| humidifier inlet | 5002 |
| humidifier outlet | 5004 |
| humidifier base | 5006 |
| reservoir | 5110 |
| humidifier reservoir | 5110 |
| water reservoir | 5110 |
| conductive portion | 5120 |
| humidifier reservoir dock | 5130 |
| locking lever | 5135 |
| water level indicator | 5150 |
| heating element | 5240 |
| air pressure | 6000 |

## Claims

1. A patient interface (3000) comprising:
a plenum chamber (3200) pressurisable to a therapeutic pressure of at least 6 cmH₂O above ambient air pressure, the plenum chamber (3200) being constructed of a relatively flexible material, the plenum chamber (3200) having a plenum chamber opening (3202), and the plenum chamber (3200) having a first connection portion (3900) positioned proximal to the plenum chamber opening (3202);
a seal-forming structure (3100) constructed and arranged to seal with a region of the patient's face surrounding an entrance to the patient's airways, said seal-forming structure (3100) having a hole (3101) therein such that the flow of air at said therapeutic pressure is delivered to at least an entrance to the patient's nares, the seal-forming structure (3100) being joined to the plenum chamber (3200) to maintain said therapeutic pressure in the plenum chamber (3200) throughout the patient's respiratory cycle in use;
a chassis (3500) constructed from a flexible material and including an inlet port (3600) sized and structured to receive a flow of air at the therapeutic pressure and direct the flow of air into the plenum chamber (3200) through the plenum chamber opening (3202) for breathing by a patient, a second connection portion (3902) positioned on the chassis (3500) and configured to releasably connect to the first connection portion (3900), a rigidity of the first connection portion (3900) and the second connection portion (3902), when connected, being increased due to the connecting, and when the first connection portion (3900) and the second connection portion (3902) are connected, the combined connection portions are more rigid than the chassis (3500) and the plenum chamber (3200);
a sealing lip (3800) positioned radially around the plenum chamber opening (3202) to seal between the plenum chamber (3200) and the chassis (3500);
a positioning and stabilising structure (3300) to provide a force to hold the seal-forming structure (3100) in a therapeutically effective position on the patient's head, the positioning and stabilising structure (3300) comprising a tie; and
a vent structure (3400) having a plurality of holes to allow a continuous flow of gases exhaled by the patient from an interior of the plenum chamber (3200) to ambient throughout the patient's respiratory cycle, said vent structure (3400) being sized and shaped to maintain the therapeutic pressure in the plenum chamber (3200) in use; and
wherein the patient interface (3000) is configured to leave the patient's mouth uncovered, or if the seal-forming structure (3100) is configured to seal around the patient's nose and mouth, the patient interface (3000) is configured to allow the patient to breath from ambient in the absence of a flow of pressurised air through the plenum chamber inlet port.

2. The patient interface (3000) of claim 1, wherein flexible material is a textile.

3. The patient interface (3000) of claim 1 or 2, wherein the first connection portion (3900) is one of a hook material and a loop material and the second connection portion (3902) is the other of the hook material and the loop material.

4. The patient interface (3000) of one of claims 1 to 3, wherein the first connection portion (3900) is positioned radially outward of the plenum chamber opening (3202), and
wherein the second connection portion (3902) is positioned radially inward of a peripheral edge (3501) of the chassis (3500).

5. The patient interface (3000) of one of claims 1 to 4, wherein the sealing lip (3800) is fixed to the chassis (3500) or the plenum chamber (3200).

6. The patient interface (3000) of one of claims 1 to 5, wherein the sealing lip (3800) is positioned radially outward or radially inward of the first connection portion (3900) and the second connection portion (3902).

7. The patient interface (3000) of one of claims 1 to 6, wherein the first connection portion (3900) and the second connection portion (3902) are continuous around the plenum chamber (3200) and the chassis (3500), respectively.

8. The patient interface (3000) of one of claims 1 to 7, wherein one of the first connection portion (3900) and the second connection portion (3902) comprise a plurality of discontinuous portions positioned around the plenum chamber (3200) and the chassis (3500), respectively, and the other of the first connection portion (3900) and the second connection portion (3902) is continuous around the plenum chamber (3200) and the chassis (3500), respectively.

9. The patient interface (3000) of one of claims 1 to 8, wherein each of the first connection portion (3900) and the second connection portion (3902) comprise a plurality of discontinuous portions positioned around the plenum chamber (3200) and the chassis (3500), respectively,
wherein the plurality of discontinuous portions of each of the first connection portion (3900) and the second connection portion (3902) are positioned at corresponding positions around the plenum chamber (3200) and the chassis (3500), respectively, to connect to one another, and
wherein the plurality of discontinuous portions of each of the first connection portion (3900) and the second connection portion (3902) are positioned asymmetrically or symmetrically around the plenum chamber (3200) and the chassis (3500), respectively.

10. The patient interface (3000) of one of claims 1 to 9, wherein the sealing lip (3800), in an undeformed state, has a dimension extending from the plenum chamber (3200) or the chassis (3500) in a direction that is orthogonal to a surface to which the sealing lip (3800) is attached, and
wherein the dimension is greater than a combined thickness of the first connection portion (3900) and the second connection portion (3902).

11. The patient interface (3000) of one of claims 1 to 10, wherein the chassis (3500) includes the vent structure (3400), or
wherein the patient interface (3000) further comprises an elbow configured to rotatably connect to the chassis (3500) at the inlet port (3600) at a first end and to an air circuit at a second end to direct the flow of air into the plenum chamber (3200), and the elbow includes the vent structure (3400).

12. The patient interface (3000) of one of claims 1 to 11, wherein the plenum chamber (3200) and the seal-forming structure (3100) are formed in one piece from a homogeneous material, a textile, or silicone.

13. The patient interface (3000) of one of claims 1 to 12, wherein the sealing lip (3800) is integrally molded to the plenum chamber (3200) or the chassis (3500).

14. The patient interface (3000) of one of claims 1 to 13, wherein the first connection portion (3900) is joined to the plenum chamber (3200) with adhesive,
wherein the plenum chamber (3200) is overmolded to the first connection portion (3900), or
wherein the first connection portion (3900) is molded into the plenum chamber (3200).

15. The patient interface (3000) of one of claims 1 to 14, wherein the sealing lip (3800) is positioned such that an increase in pressure within the plenum chamber (3200) increases a sealing force of the sealing lip (3800) between the plenum chamber (3200) and the chassis (3500).

## Patentansprüche

1. Patientenschnittstelle (3000), aufweisend:
eine Luftkammer (3200), die auf einen therapeutischen Druck von mindestens 6 cmH₂O über dem Umgebungsluftdruck gebracht werden kann, wobei die Luftkammer (3200) aus einem relativ flexiblen Material aufgebaut ist; die Luftkammer (3200) eine Luftkammeröffnung (3202) aufweist und die Luftkammer (3200) einen ersten Verbindungsabschnitt (3900) aufweist, der proximal zur Luftkammeröffnung (3202) positioniert ist;
eine dichtungsbildende Struktur (3100), die zum Bilden einer Dichtung mit einem Bereich des Gesichts des Patienten, der einen Eingang zu den Atemwegen des Patienten umgibt, aufgebaut und angeordnet ist, wobei die dichtungsbildende Struktur (3100) ein Loch (3101) darin aufweist, so dass der Luftstrom mit dem therapeutischen Druck mindestens einem Eingang zu den Nasenlöchern des Patienten zugeführt wird, wobei die dichtungsbildende Struktur (3100) mit der Luftkammer (3200) verbunden ist, um im Gebrauch den therapeutischen Druck in der Luftkammer (3200) während des gesamten Atemzyklus des Patienten aufrecht zu erhalten;
ein Chassis (3500), das aus einem flexiblen Material aufgebaut ist und einen Einlassanschluss (3600), der zum Aufnehmen eines Luftstroms mit dem therapeutischen Druck und zum Leiten des Luftstroms in die Luftkammer (3200) durch die Luftkammeröffnung (3202) zum Atmen durch einen Patienten bemessen und ausgebildet ist, einen zweiten Verbindungsabschnitt (3902), der auf dem Chassis (3500) positioniert und zum lösbaren Verbinden mit dem ersten Verbindungsabschnitt (3900) konfiguriert ist, aufweist, wobei eine Steifigkeit des ersten Verbindungsabschnitts (3900) und des zweiten Verbindungsabschnitts (3902) bei deren Verbindung aufgrund der Verbindung erhöht ist und die kombinierten Verbindungsabschnitte steifer als das Chassis (3500) und die Luftkammer (3200) sind, wenn der erste Verbindungsabschnitt (3900) und der zweite Verbindungsabschnitt (3902) miteinander verbunden sind;
eine Dichtlippe (3800), die radial um die Luftkammeröffnung (3202) positioniert ist, um zwischen der Luftkammer (3200) und dem Chassis (3500) eine Dichtung zu bilden;
eine Positionierungs- und Stabilisierungsstruktur (3300), die eine Kraft zum Halten der dichtungsbildenden Struktur (3100) in einer therapeutisch wirksamen Position auf dem Kopf des Patienten bereitstellt, wobei die Positionierungs- und Stabilisierungsstruktur (3300) ein Bindeelement aufweist; und
eine Entlüftungsstruktur (3400) mit mehreren Löchern, um während des gesamten Atemzyklus des Patienten einen kontinuierlichen Strom der vom Patienten ausgeatmeten Gase aus dem Inneren der Luftkammer (3200) in die Umgebung zu ermöglichen, wobei die Entlüftungsstruktur (3400) so bemessen und geformt ist, dass der therapeutische Druck in der Luftkammer (3200) im Gebrauch aufrecht erhalten wird; und
wobei die Patientenschnittstelle (3000) dazu konfiguriert ist, den Mund des Patienten unbedeckt zu lassen, oder, wenn die dichtungsbildende Struktur (3100) dazu konfiguriert ist, den Bereich um die Nase und den Mund des Patienten herum abzudichten, die Patientenschnittstelle (3000) dazu konfiguriert ist, dem Patienten zu ermöglichen, bei Abwesenheit eines Stroms von unter Druck stehender Luft durch den Einlassanschluss der Luftkammer aus der Umgebung zu atmen.

2. Patientenschnittstelle (3000) nach Anspruch 1, wobei das flexible Material ein Textilmaterial ist.

3. Patientenschnittstelle (3000) nach Anspruch 1 oder 2, wobei der erste Verbindungsabschnitt (3900) eines von einem Hakenmaterial oder einem Schlaufenmaterial und der zweite Verbindungsabschnitt (3902) dann das jeweils andere von dem Hakenmaterial oder dem Schlaufenmaterial ist.

4. Patientenschnittstelle (3000) nach einem der Ansprüche 1 bis 3, wobei der erste Verbindungsabschnitt (3900) radial außerhalb der Luftkammer (3202) positioniert ist und.
wobei der zweite Verbindungsabschnitt (3902) radial innerhalb einer Umfangskante (3501) des Chassis (3500) positioniert ist.

5. Patientenschnittstelle (3000) nach einem der Ansprüche 1 bis 4, wobei die Dichtlippe (3800) am Chassis (3500) oder an der Luftkammer (3200) befestigt ist.

6. Patientenschnittstelle (3000) nach einem der Ansprüche 1 bis 5, wobei die Dichtlippe (3800) radial außerhalb oder radial innerhalb des ersten Verbindungsabschnitts (3900) und des zweiten Verbindungsabschnitts (3902) positioniert ist.

7. Patientenschnittstelle (3000) nach einem der Ansprüche 1 bis 6, wobei der erste Verbindungsabschnitt (3900) und der zweite Verbindungsabschnitt (3902) durchgehend um die Luftkammer (3200) bzw. das Chassis (3500) verlaufen.

8. Patientenschnittstelle (3000) nach einem der Ansprüche 1 bis 7, wobei einer von erstem Verbindungsabschnitt (3900) und zweitem Verbindungsabschnitt (3902) mehrere um die Luftkammer (3200) bzw. das Chassis (3500) herum positionierte nicht durchgehende Abschnitte aufweist und der jeweils andere vom erstem Verbindungsabschnitt (3900) und zweitem Verbindungsabschnitt (3902) durchgehend um die Luftkammer (3200) bzw. das Chassis (3500) verläuft.

9. Patientenschnittstelle (3000) nach einem der Ansprüche 1 bis 8, wobei der erste Verbindungsabschnitt (3900) und der zweite Verbindungsabschnitt (3902) jeweils mehrere nicht durchgehende Abschnitte aufweisen, die um die Luftkammer (3200) bzw. das Chassis (3500) herum positioniert sind,
wobei die mehreren nicht durchgehenden Abschnitte des ersten Verbindungsabschnitts (3900) und des zweiten Verbindungsabschnitts (3902) an entsprechenden Positionen um die Luftkammer (3200) bzw. das Chassis (3500) herum positioniert sind, um miteinander verbunden zu werden, und
wobei die mehreren nicht durchgehenden Abschnitte des ersten Verbindungsabschnitts (3900) und des zweiten Verbindungsabschnitts (3902) asymmetrisch oder symmetrisch um die Luftkammer (3200) bzw. das Chassis (3500) herum positioniert sind.

10. Patientenschnittstelle (3000) nach einem der Ansprüche 1 bis 9, wobei die Dichtlippe (3800) in einem nicht verformten Zustand eine Abmessung aufweist, die sich von der Luftkammer (3200) oder dem Chassis (3500) orthogonal zu einer Fläche erstreckt, an der die Dichtlippe (3800) angebracht ist, und
wobei die Abmessung größer als die kombinierte Dicke des ersten Verbindungsabschnitts (3900) und des zweitem Verbindungsabschnitts (3902) ist.

11. Patientenschnittstelle (3000) nach einem der Ansprüche 1 bis 10, wobei das Chassis (3500) die Entlüftungsstruktur (3400) aufweist oder
wobei die Patientenschnittstelle (3000) ferner ein Winkelstück aufweist, das zum drehbaren Verbinden mit dem Chassis (3500) am Einlassanschluss (3600) an einem ersten Ende und mit einer Luftleitung an einem zweiten Ende konfiguriert ist, um den Luftstrom in die Luftkammer (3200) zu leiten, wobei das Winkelstück die Entlüftungsstruktur (3400) aufweist.

12. Patientenschnittstelle (3000) nach einem der Ansprüche 1 bis 11, wobei die Luftkammer (3200) und die dichtungsbildende Struktur (3100) in einem Stück aus einem homogenen Material, einem Textilmaterial oder Silikon gebildet sind.

13. Patientenschnittstelle (3000) nach einem der Ansprüche 1 bis 12, wobei die Dichtlippe (3800) integral an die Luftkammer (3200) oder das Chassis (3500) angeformt ist.

14. Patientenschnittstelle (3000) nach einem der Ansprüche 1 bis 13, wobei der erste Verbindungsabschnitt (3900) mit Klebstoff mit der Luftkammer (3200) verbunden ist,
wobei die Luftkammer (3200) auf den ersten Verbindungsabschnitt (3900) aufgeformt ist; oder
wobei der erste Verbindungsabschnitt (3900) in die Luftkammer (3200) eingeformt ist.

15. Patientenschnittstelle (3000) nach einem der Ansprüche 1 bis 14, wobei die Dichtlippe (3800) so positioniert ist, dass eine Erhöhung des Drucks in der Luftkammer (3200) eine Dichtungskraft der Dichtlippe (3800) zwischen der Luftkammer (3200) und dem Chassis (3500) erhöht.

## Revendications

1. Interface patient (3000), comprenant :
une chambre de distribution (3200) pouvant être mise sous pression à une pression thérapeutique d'au moins 6 cmH₂O au-dessus de la pression d'air ambiant, la chambre de distribution (3200) étant constituée d'un matériau relativement flexible, la chambre de distribution (3200) comportant une ouverture de chambre de distribution (3202), et la chambre de distribution (3200) comportant une première partie de liaison (3900) positionnée proximale à l'ouverture de chambre de distribution (3202) ;
une structure de formation d'étanchéité (3100) structurée et conçue pour établir une étanchéité avec une région du visage du patient entourant une entrée des voies aériennes du patient, ladite structure de formation d'étanchéité (3100) comportant un trou (3101) en son sein de sorte que le flux d'air à ladite pression thérapeutique est délivré à au moins une entrée des narines du patient, la structure de formation d'étanchéité (3100) étant reliée à la chambre de distribution (3200) pour maintenir ladite pression thérapeutique dans la chambre de distribution (3200) sur tout le cycle respiratoire du patient en utilisation ;
un châssis (3500) constitué d'un matériau flexible et comprenant un orifice d'entrée (3600) dimensionné et structuré pour recevoir un flux d'air à la pression thérapeutique et pour diriger le flux d'air dans la chambre de distribution (3200) par l'intermédiaire de l'ouverture de chambre de distribution (3202) à des fins de respiration par un patient, une seconde partie de liaison (3902) positionnée sur le châssis (3500) et configurée pour être liée de manière libérable à la première partie de liaison (3900), une rigidité de la première partie de liaison (3900) et de la seconde partie de liaison (3902), lorsqu'elles sont liées, étant augmentée en raison de la liaison, et lorsque la première partie de liaison (3900) et la seconde partie de liaison (3902) sont liées, les parties de liaison combinées sont plus rigides que le châssis (3500) et la chambre de distribution (3200) ;
une lèvre d'étanchéité (3800) positionnée radialement autour de l'ouverture de chambre de distribution (3202) pour établir une étanchéité entre la chambre de distribution (3200) et le châssis (3500) ;
une structure de positionnement et de stabilisation (3300) pour établir une force servant à maintenir la structure de formation d'étanchéité (3100) dans une position thérapeutiquement efficace sur la tête du patient, la structure de positionnement et de stabilisation (3300) comprenant une attache ; et
une structure d'évent (3400) comportant une pluralité de trous servant à permettre un écoulement continu de gaz exhalés par le patient de l'intérieur de la chambre de distribution (3200) vers l'environnement ambiant sur tout le cycle respiratoire du patient, ladite structure d'évent (3400) étant dimensionnée et formée pour maintenir la pression thérapeutique dans la chambre de distribution (3200) en utilisation ; et
dans laquelle l'interface patient (3000) est configurée pour laisser la bouche du patient découverte, ou si la structure de formation d'étanchéité (3100) est configurée pour établir une étanchéité autour du nez et de la bouche du patient, l'interface patient (3000) est configurée pour permettre au patient de respirer à partir de l'environnement ambiant en l'absence d'un flux d'air sous pression à travers l'orifice d'entrée de chambre de distribution.

2. Interface patient (3000) selon la revendication 1, dans laquelle le matériau flexible est un textile.

3. Interface patient (3000) selon la revendication 1 ou la revendication 2, dans laquelle la première partie de liaison (3900) est l'un d'un matériau à crochets et d'un matériau à boucles et la seconde partie de liaison (3902) est l'autre du matériau à crochets et du matériau à boucles.

4. Interface patient (3000) selon l'une des revendications 1 à 3, dans laquelle la première partie de liaison (3900) est positionnée radialement vers l'extérieur de l'ouverture de chambre de distribution (3202), et
dans laquelle la seconde partie de liaison (3902) est positionnée radialement vers l'intérieur d'un bord périphérique (3501) du châssis (3500).

5. Interface patient (3000) selon l'une des revendications 1 à 4, dans laquelle la lèvre d'étanchéité (3800) est fixée au châssis (3500) ou à la chambre de distribution (3200).

6. Interface patient (3000) selon l'une des revendications 1 à 5, dans laquelle la lèvre d'étanchéité (3800) est positionnée radialement vers l'extérieur ou radialement vers l'intérieur de la première partie de liaison (3900) et de la seconde partie de liaison (3902).

7. Interface patient (3000) selon l'une des revendications 1 à 6, dans laquelle la première partie de liaison (3900) et la seconde partie de liaison (3902) sont continues autour, respectivement, de la chambre de distribution (3200) et du châssis (3500).

8. Interface patient (3000) selon l'une des revendications 1 à 7, dans laquelle l'une de la première partie de liaison (3900) et de la seconde partie de liaison (3902) comprend une pluralité de parties discontinues positionnées autour, respectivement, de la chambre de distribution (3200) et du châssis (3500), et l'autre de la première partie de liaison (3900) et de la seconde partie de liaison (3902) est continue autour, respectivement, de la chambre de distribution (3200) et du châssis (3500).

9. Interface patient (3000) selon l'une des revendications 1 à 8, dans laquelle chacune de la première partie de liaison (3900) et de la seconde partie de liaison (3902) comprend une pluralité de parties discontinues positionnées autour, respectivement, de la chambre de distribution (3200) et du châssis (3500),
dans laquelle les parties de la pluralité de parties discontinues de chacune de la première partie de liaison (3900) et de la seconde partie de liaison (3902) sont positionnées à des positions correspondantes autour, respectivement, de la chambre de distribution (3200) et du châssis (3500) pour être liées les unes aux autres, et
dans laquelle les parties de la pluralité de parties discontinues de chacune de la première partie de liaison (3900) et de la seconde partie de liaison (3902) sont positionnées de manière asymétrique ou symétrique autour, respectivement, de la chambre de distribution (3200) et du châssis (3500).

10. Interface patient (3000) selon l'une des revendications 1 à 9, dans laquelle la lèvre d'étanchéité (3800), dans un état non déformé, a une dimension s'étendant de la chambre de distribution (3200) ou du châssis (3500) dans une direction qui est orthogonale à une surface à laquelle est fixée la lèvre d'étanchéité (3800), et
dans laquelle la dimension est supérieure à une épaisseur combinée de la première partie de liaison (3900) et de la seconde partie de liaison (3902).

11. Interface patient (3000) selon l'une des revendications 1 à 10, dans laquelle le châssis (3500) comprend la structure d'évent (3400), ou
dans laquelle l'interface patient (3000) comprend en outre
un coude configuré pour être raccordé tournant au châssis (3500) au niveau de l'orifice d'entrée (3600) au niveau d'une première extrémité et à un circuit d'air au niveau d'une seconde extrémité pour diriger le flux d'air dans la chambre de distribution (3200), et le coude comprend la structure d'évent (3400).

12. Interface patient (3000) selon l'une des revendications 1 à 11, dans laquelle la chambre de distribution (3200) et la structure de formation d'étanchéité (3100) sont formées d'une seule pièce à partir d'un matériau homogène, d'un textile, ou d'une silicone.

13. Interface patient (3000) selon l'une des revendications 1 à 12, dans laquelle la lèvre d'étanchéité (3800) est moulée d'un seul tenant avec la chambre de distribution (3200) ou le châssis (3500).

14. Interface patient (3000) selon l'une des revendications 1 à 13, dans laquelle la première partie de liaison (3900) est reliée à la chambre de distribution (3200) par un adhésif,
dans laquelle la chambre de distribution (3200) est surmoulée sur la première partie de liaison (3900), ou
dans laquelle la première partie de liaison (3900) est moulée dans la chambre de distribution (3200).

15. Interface patient (3000) selon l'une des revendications 1 à 14, dans laquelle la lèvre d'étanchéité (3800) est positionnée de sorte qu'une augmentation de pression à l'intérieur de la chambre de distribution (3200) augmente une force d'étanchéité de la lèvre d'étanchéité (3800) entre la chambre de distribution (3200) et le châssis (3500).
